# EUROPEAN PATENT APPLICATION

(11) **EP 3 272 752 A1**
(43) Date of publication of application: **24.01.2018**
(21) Application number: 16764117.4
(22) Date of filing: 18.03.2016
(51) Int. Cl.: C07D 487/04, A61K 31/519, A61K 45/06, A61P 37/02, A61P 29/00, A61P 5/14, A61P 3/10, A61P 21/04, A61P 1/04, A61P 13/12, A61P 1/16, A61P 25/00

(54) **OPTIMISED COMBINATION THERAPY AND USE THEREOF TO TREAT CANCER AND AUTOIMMUNE DISEASE**

(30) Priority: 19.03.2015 CN 201510119944
(71) Applicant: Zhejiang DTRM Biopharma Co., Ltd., Hangzhou, Zhejiang 311400 (CN)
(72) Inventor: HE, Wei, Shanghai 200233 (CN)
(74) Representative: Evenson, Jane Harriet
(86) International application number: PCT/CN2016/000149
(87) International publication number: WO 2016/145935

(57) **Abstract**

Provided is a series of new poly-fluorosubstituted pyrazolopyrimidine compounds or salts. The compounds are Bruton's tyrosine kinase (BTK) inhibitors. The compounds have better kinase inhibition selectivity and pharmokinetic properties. Also provided is a preparation method for the compounds. Also provided is a combination therapy including the compounds in combination with another targeted drug composition or another drug. The optimised combination therapy has a cooperative effect, inhibits the existence of a tumour better than a single targeted drug, and causes certain tumours to completely disappear. The optimised combination therapy treats drug resistance and cancer recurrence of a tumour better than a single targeted drug, and the treatment cycle is shorter. The present invention also relates to a combined compound and a pharmaceutical preparation wherein said combined compound acts as an active ingredient, said pharmaceutical preparation being safer at a lower dosage and having a cooperative effect efficacy. Also provided is a method of using the compounds and a preparation thereof to treat and inhibit an autoimmune disease or illness, a heterogeneous autoimmune disease or illness, an inflammatory disease, a cancer or an illness.

## Description

### FIELD OF THE INVENTION

The invention relates to a novel series of multi-fluoro-substituted pyrazolopyrimidine compounds and the synthesis methods, as well as pharmaceutical compositions comprising the compounds described herein as active ingredients and the methods of inhibition of Bruton's tyrosine kinase activity. The invention also relates to optimized combinations of various drugs for the inhibition of *in vitro* tumor cell viability and *in vivo* tumors. Optimized combinations have a synergistic effect, which could inhibit tumor survival more effectively than single-target agents and cause certain tumors to disappear completely. Compared with single-target agents, optimized combinations can better avoid drug resistance and cancer recurrence. Optimized combinations are safer due to lower dose and can shorten treatment cycles because of better therapeutic effects.

### BACKGROUND OF THE INVENTION

Anticancer treatment has been developed from toxic chemotherapy to combination chemotherapy (e.g., CHOP), antibodies (e.g., rituximab), combination of chemotherapy and antibody therapy (R-CHOP), and the exciting anticancer immune therapy (PD-1 and PD-L1 antibody) and a chimeric antigen receptor T cell therapy (CAR-T). The patient's life and life quality has been improved, however, due to the complexity of cancer, mutations in a single pathway or signaling pathways can lead to ineffectual treatment or recurrence of disease. Small molecule targeted therapy requires long-term daily administration, and if the drug administration is stopped, rebound or recurrence of cancer may occur. The delivery methods of antibody and chemotherapy are complex. The combination of antibody and chemotherapy may have additive or synergistic effects, but rarely results in curing the cancer. Combination therapies of targeted therapy with immune anticancer therapy or chimeric antigen receptor T-cell immunotherapy (CAR-T) are developing, but may have safety concerns. Oral therapy has many advantages, for example, we can immediately stop the administration if serious side effects occur. For antibody and cell therapy, it is difficult to control serious side effects. At present, single-target therapy or combination therapy of targeting two pathways have some limitations, and therefore the clinical efficacy of cancer treatment (overall response rate, ORR) are mostly partial response (PR) or stable disease (SD), and rarely complete response (CR). These treatments usually prolong a patient's life by only a few months. In addition, the complex cell therapy or the combination of antibody with chemotherapy may have safety issues. What is more, these treatments are limited to the hospital setting. These aforementioned disadvantages not only lead to high costs for cancer treatment, but also affect the patient's wishes for treatment as well as treatment compliance, and are an obstacle in expanding cancer treatment to the majority of patients. Furthermore, the price for targeting single-pathway therapy is US$100,000-200,000 per patient, and the price of combination therapies will be doubled or even higher. The development of these expensive treatments is unsustainable. Therefore, both patients and the market have a clear need to seek a better combination therapy. The new combination therapy is expected to be more effective with fewer side effects and a shorter treatment cycle. With the assistance of a suitable companion diagnostic, optimized combination therapy (personalized medicine or precise medicine) might cure cancer subtypes, or allow for better control of drug resistance.

Ibrutinib, the first Bruton's tyrosine kinase (BTK) inhibitor, was approved by the U.S. Food and Drug administration for the treatment of mantle cell lymphoma (MCL) and chronic lymphocytic leukemia (CLL) and showed good effect, but the clinical treatment has produced C481s and other mutations that cause drug resistance (Furman et al: Ibrutinib resistance in chronic lymphocytic leukemia, N Engl J Med, 2014, 2352). Furthermore, the pharmacokinetics of Ibrutinib vary widely among patients (Marostica et al: Population harmacokinetic [sic] model of Ibrutinib, a Bruton tyrosine kinase inhibitor, in patients with B cell malignancies, Cancer Chemother Pharmacol (2015) 75: 111-121), a toxicokinetic study found AUC in rats and dogs are low 1000 h*ng/mL (male rats, 40mg/kg) and 3300 h*ng/mL (female rats, 40mg/kg) and 1780 h*ng/mL (male dogs, 24mg/kg) and 1850 h*ng/mL (female dogs, 24mg/kg) (FDA's NDA Application Number 205552Orig1s000_pharmacological review). BTK plays a crucial role in the B-cell signaling pathway linking B-cell receptor (BCR) simulation on the cell surface to downstream intracellular responses. BTK is a key regulator of B-cell development, activation, proliferation, and survival.

Everolimus, a mammalian target of rapamycin (mTOR) kinase inhibitor, was approved by the U.S. Food and Drug Administration for the treatment of breast cancer, pancreatic cancer, renal cell carcinoma, renal angiomyolipoma, and tuberous sclerosis. The mechanism of mTOR protein has been clearly elucidated. mTOR is a key regulator of cell growth, proliferation, metabolism, and apoptosis in complex signaling pathways. In addition, Everolimus is used to treat organ transplant rejection at low doses, as organ transplant also activates mTOR.

Pomalidomide, an immunomodulatory drug (IMiD), was approved by the U.S. Food and Drug Administration in 2013 for the treatment of multiple myeloma (MM). Pomalidomide and its analog IMiDs inhibit cell factors TNF-α, IL-1β, IL-6, IL-12, and GM-CSF. IMiDs possess anti-angiogenic, anti-proliferative, and pro-apoptotic properties, and they also stimulate T lymphocyte to induce T cell proliferation, T cell cytokine production, and T cell cytotoxicity, thus increasing the T cells' anti-cancer activity.

Venetoclax or ABT-199 is a novel and specific inhibitor of B-cell lymphoma-2 (Bcl-2) in clinical studies. Bcl-2 protein plays a key role in apoptosis. ABT-199 once triggered tumor lysis syndrome (TLS) and resulted in the death of a patient in clinical trial.

Methotrexate (MTX) is an antifolate antineoplastic drug and rheumatoid arthritis drug, mainly through the inhibition of dihydrofolate reductase to block cell synthesis and inhibit cell growth and proliferation.

BTK is a member of the Tec family of protein tyrosine kinases. BTK consists of unique N-terminal domain, i.e., pleckstrin homology (PH), Tec homology (TH) homology region, Src homology 3 (SH3) domains, Src homology 2 (SH2) domain and a catalytic domain, and tyrosine kinase or Src homology 1 (TK or SHI) domain of the kinase domains (Akinleye et al: Ibrutinib and novel BTK inhibitors in clinical development, Journal of Hematology & Oncology 2013, 6:59). In the normal development of B lymphocytes, the correct expression of BTK gene in different regions plays a key role in the function of B cells and various signal transduction pathways.

BTK functions downstream of multiple receptors including growth factors, B-cell antigen, chemokine, and innate immune receptors, and thereby initiates a diverse range of cellular processes, such as cell proliferation, survival, differentiation, motility, angiogenesis, cytokine production, and antigen presentation. Therefore, BTK plays an important role in many hematopoietic cell signaling pathways, and it is also crucial in B cell activation, development, survival, and signal transduction (Kurosaki, Molecular mechanisms in B cell antigen receptor signaling. Curr OP Imm, 1997, 9(3): 309-18).

There is evidence to prove that B cells have immune regulatory effects on their immune responses and inflammatory responses. For example, CD20 antibody rituximab (rituxan) is a treatment agent based on the protein by consumption of B cell and used as a treatment of autoimmune diseases, such as chronic lymphocytic leukemia and inflammatory diseases, such as rheumatoid arthritis. Therefore, protein kinase, which plays a key role in the activation of B cells, will be helpful for B cell related diseases.

Evidence for the role of BTK in autoimmune diseases has been provided by the BTK-deficient mice and the BTK-sufficient mice model (Kil LP, et al: Bruton's tyrosine kinase mediated signaling enhances leukemogenesis in a mouse model for chronic lymphocytic leukemia. Am J Blood Res 2013, 3(1): 71-83.). In a mouse model of chronic lymphocytic leukemia (CLL), BTK-deficient mice completely stopped chronic lymphocytic leukemia, BTK overexpression accelerated leukemia, increasing mortality.

The selectivity of the known BTK inhibitor is not ideal: not only inhibiting BTK, but also various other kinases (such as ETK, EGF, BLK, FGR, HCK, YES, BRK, and JAK3, etc.), which may cause more side effects. Better selective inhibitors may result in fewer side effects.

The known BTK inhibitors produce a variety of derivatives, which also affect the efficacy and side effects. It is also known that the pharmacokinetics of BTK inhibitors can also be improved.

### DESCRIPTION OF THE INVENTION

The invention relates to the therapeutic methods that include BTK inhibitors to treat or inhibit autoimmune disease, hypersensitivity disease, inflammatory diseases and cancer. Effective doses for patients described herein include compounds that have a structure of Formula (I), (II), (Ia), (IIa), (Ib) and (IIb) or pharmaceutically acceptable salts thereof. 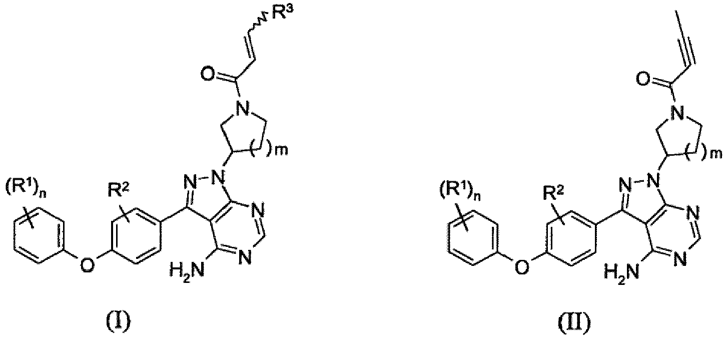 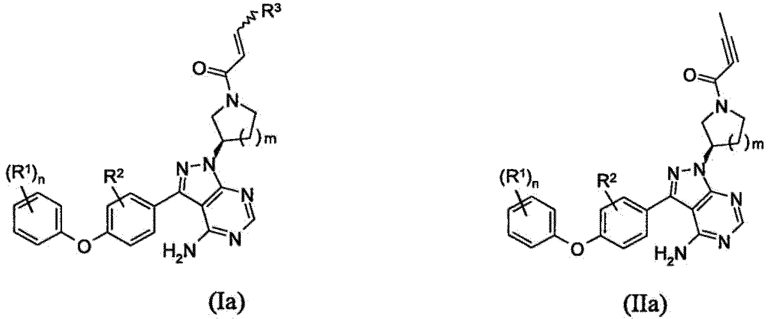 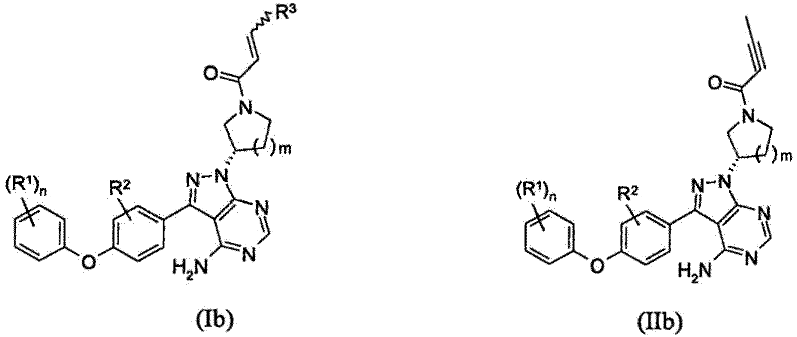 Wherein:
R¹ is Fluorine; n is 1, 2, 3 or 4;
R² is Fluorine; m is 1 or 2
R³ is Hydrogen or Deuterium

The term "pharmaceutically acceptable salt" refers to salts formed with an acid or a base, including, but not limited to, (a) acid addition salts: inorganic acids (e.g., hydrochloric acid, hydrobromic acid, sulfuric acid, phosphoric acid, nitric acid and other organic acids), and organic acids (e.g., acetic acid, oxalic acid, tartaric acid, succinic acid, malic acid, ascorbic acid, benzoic acid, tannic acid, pamoic acid, alginic acid, poly-glutamic acid, and salicylic acid); (b) base addition salts, formed with metal cations, such as zinc, calcium, sodium, potassium, etc.

### Synthesis Scheme

The present invention exemplifies examples and embodiments disclosed herein. The particular embodiment of the present invention is selected from the group of disclosed embodiments and their pharmaceutically-acceptable salts thereof and their individual diastereomers or salts thereof.

The invention of synthesis methods has been actively explored. A novel method for the synthesis of pyrazolopyrimidine compounds was successfully designed (see Schemes 1-2 and the specific reaction instance).

Unless otherwise specified, in the following reaction schemes and discussion, R1, R2, R3, m, n have the same meaning as defined above.

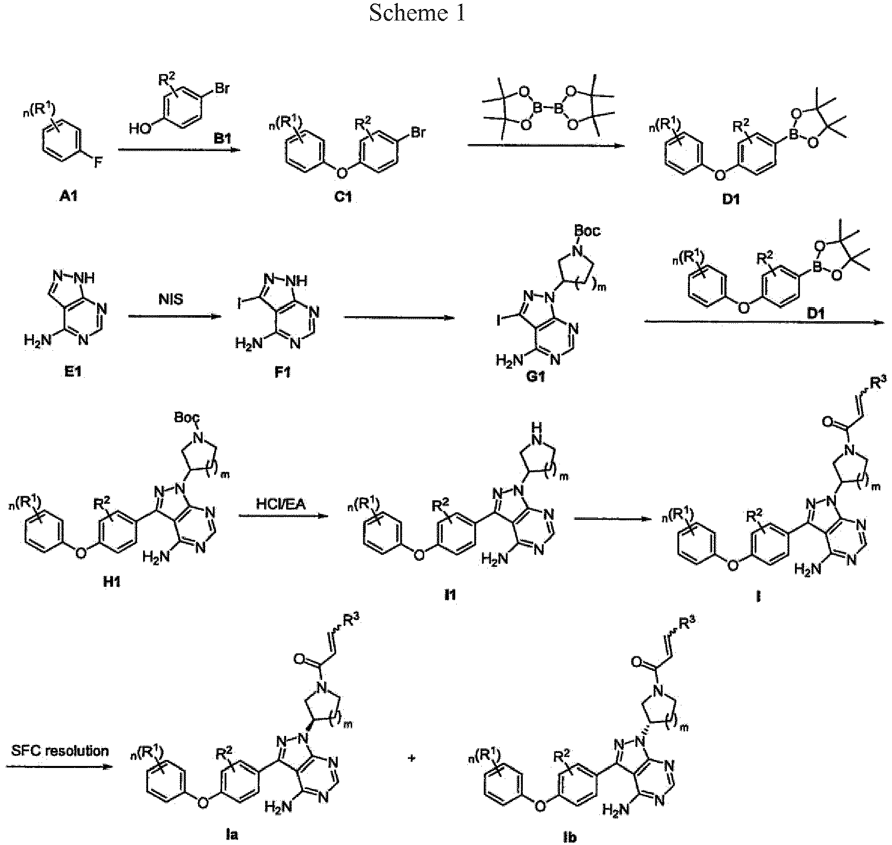

Fluoro-substituted starting material A1 was treated with substituted phenol B1 to generate intermediate C1 under basic condition (e.g., potassium carbonate) in a suitable solvent (e.g., DMF). Intermediate C1 then reacted with bis(pinacolato)diboron to give intermediate D1 with a suitable catalyst (e.g., [1,1'-bis(diphenylphosphino)ferrocene]dichloropalladium(II)) under basic condition (e.g., potassium acetate) in a suitable solvent (e.g., 1,4-dioxane). Iodination of 1H- pyrazolo[3,4-d]pyrimidin-4-amine with NIS formed intermediate F1, followed by Mitsunobu reaction or displacement reaction to furnish intermediate G1. Intermediate G1 was treated with compound D1 above obtained to give intermediate H1 with a suitable catalyst (e.g., Pd-118) under basic condition (e.g., potassium phosphate) in a suitable solvent (e.g., 1,4-dioxane). De-Boc protection of intermediate H1 gave amine I1 under acidic condition. Intermediate I1 was reacted with an electrophilic reagent to form amide J1. If J1 is racemic, optically active compounds K1 and L1 could be obtained by SFC chiral resolution.

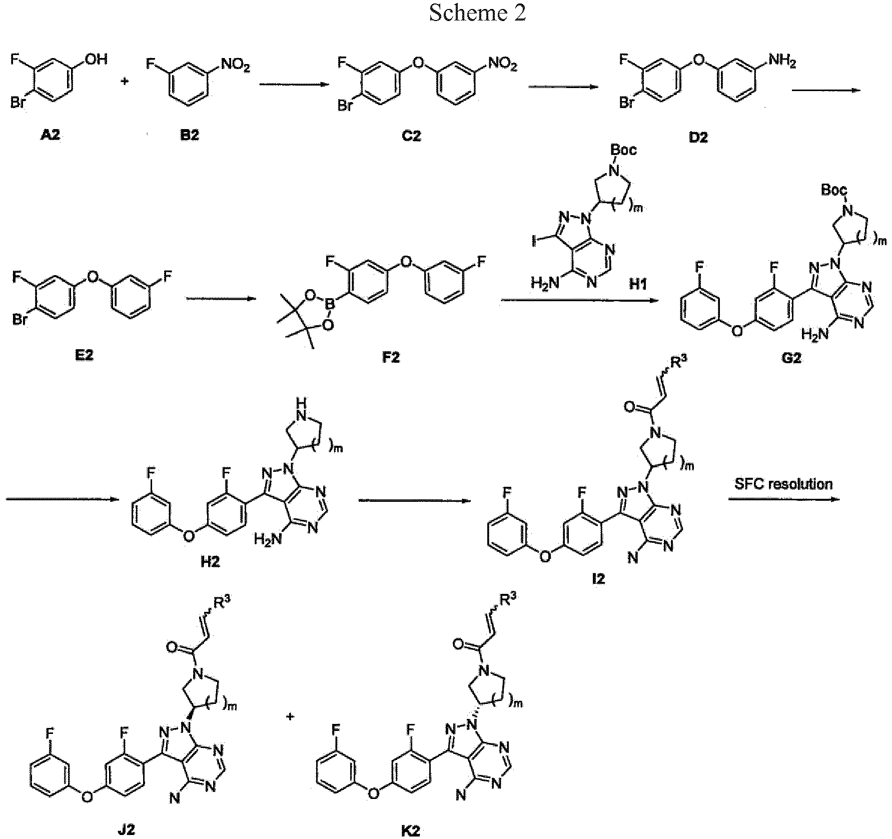

3-fluoro-4-bromophenol reacted with 1-fluoro-3-nitrobenzene to generate intermediate C2 with a base (e.g., potassium carbonate) in a suitable solvent (e.g., DMF). The obtained nitro compound C2 was reduced to the amine D2 with appropriate reducing reagents (e.g., iron powder and ammonium chloride) in appropriate solvents (e.g., ethanol and water), followed by treatment with sodium nitrite and hydrogen fluoride pyridine to generate fluoro-substituted intermediate E2. Intermediate E2 then reacted with bis(pinacolato)diboron to give intermediate F2 with a suitable catalyst (e.g., [1,1'-bis(diphenylphosphino)ferrocene]dichloropalladium(II)) under basic condition (e.g., potassium acetate) in a suitable solvent (e.g., 1,4-dioxane). Intermediate H1 was treated with compound F2 above obtained to give intermediate G2 with a suitable catalyst (e.g., Pd-118) under basic condition (e.g., potassium phosphate) in a suitable solvent (e.g., 1,4-dioxane). De-Boc protection of intermediate G2 gave amine H2 under acidic condition. Intermediate H2 was reacted with an electrophilic reagent to form amide 12. If 12 is racemic, optically active compounds J2 and K2 could be obtained by SFC chiral resolution.

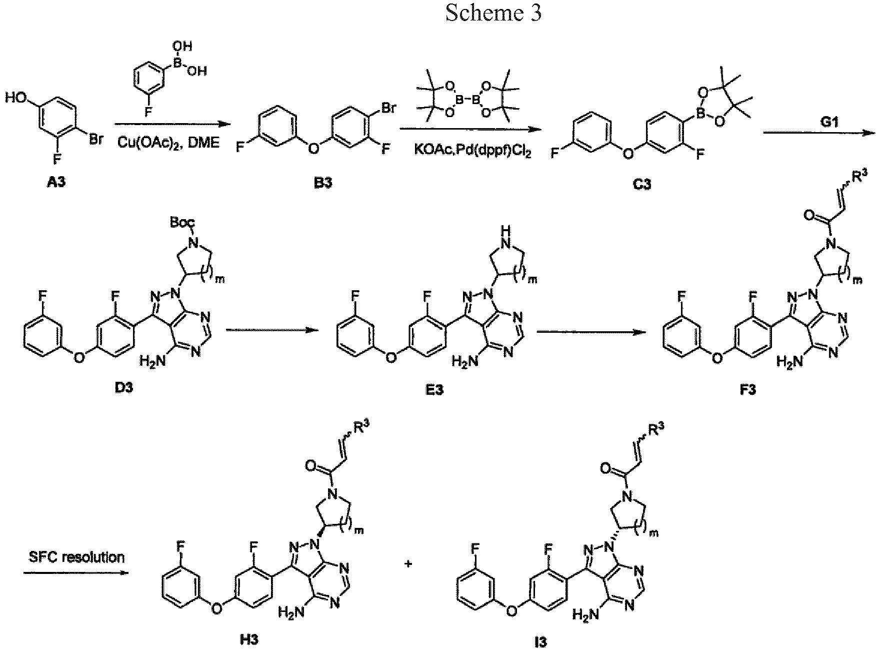

3-fluoro-4-bromophenol reacted with 3-fluorophenylboronic acid to generate intermediate B3 with an appropriate catalyst (e.g., copper acetate). Intermediate B3 then reacted with bis(pinacolato)diboron to give intermediate C3 with a suitable catalyst (e.g., [1,1'-bis(diphenylphosphino)ferrocene]dichloropalladium(II)). Intermediate G1 was treated with compound C3 above obtained to give intermediate D3 with an appropriate catalyst (e.g., [1,1'-bis(diphenylphosphino)ferrocene]dichloropalladium(II)) under basic condition (e.g., potassium acetate) in a suitable solvent (e.g., 1,4-dioxane). De-Boc protection of intermediate D3 gave amine E3 under acidic condition. Intermediate E3 was reacted with an electrophilic reagent to form amide F3. If F3 is racemic, optically active compounds H3 and I3 could be obtained by SFC chiral resolution.

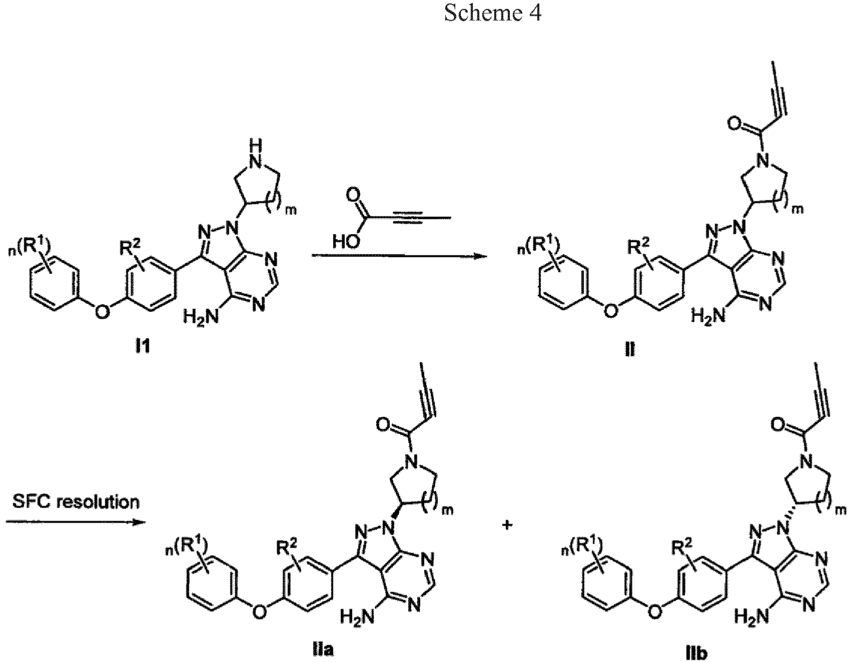

Amine compound I1 can be reacted with 2-butynoic acid to yield compound II. If compound II is racemic, optically active compounds IIa and IIb can be obtained by SFC chiral resolution.

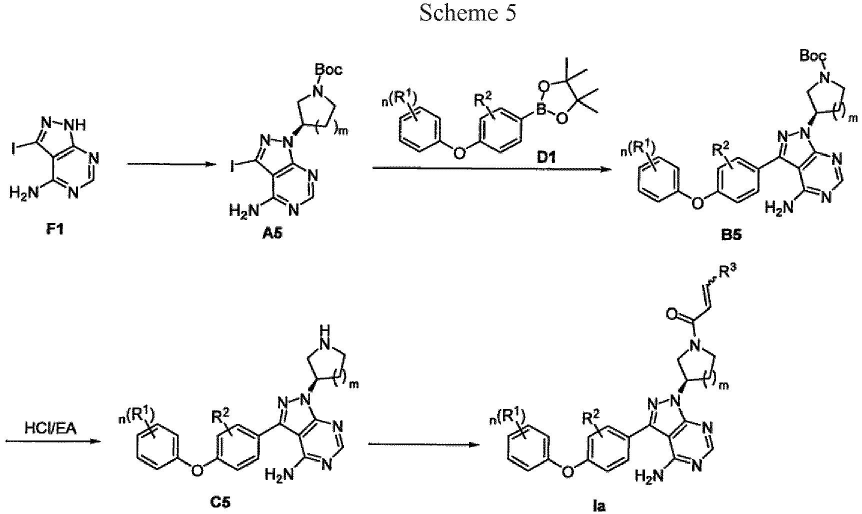

An optically active intermediate A5 was formed from the intermediate F1 via Mitsunobu reaction or substitution reaction. In the presence of a suitable base, e.g., potassium phosphate, and a suitable catalyst (e.g., Pd-118), the intermediate A5 can be reacted with boronate ester D1 in a suitable solvent, e.g., 1,4-dioxane and water, to yield the intermediate B5. Boc protection of the intermediate B5 in acidic condition yielded the amine compound C5. The amine compound A5 can be reacted with an electrophilic reagent to yield compound Ia.

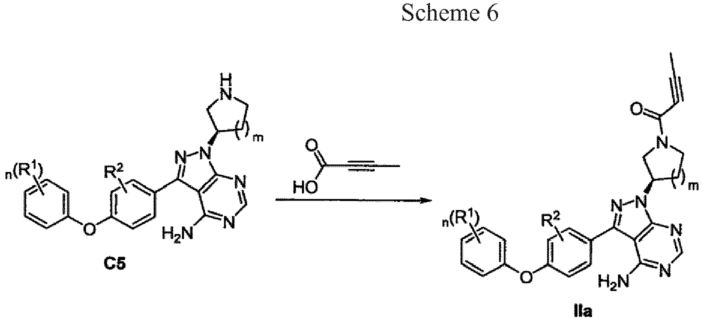

Amine compound C5 can be reacted with 2-butynoic acid to yield the compound IIa.

The present invention provides compounds having a structure shown in Formula (I), (II), (Ia), (IIa), (Ib) and (IIb), and enantiomers thereof, diastereomers thereof, or pharmaceutically acceptable salts thereof.

The compounds of Formula (I), (II), (Ia), (IIa), (Ib) and (IIb) of the present invention comprise one or more stable isotopes or radio isotopes, wherein the isotopes include, but are not limited to, ²H, ³H, ¹³C, ¹⁴C, ¹⁵N, ¹⁸O and so on.

The present invention is the first case to introduce ²H, which is an isotope of ¹H, into BTK inhibitors.

¹H, which is at the end of the double bond of the vinyl group in the compounds of Formula (I), (II), (Ia), (IIa), (Ib) and (IIb) may be replaced with ²H to reduce drug inactivation caused by oxidation/reduction of the double bond.

The present invention provides methods for preparation of the compounds represented by Formulas (I), (II), (Ia), (IIa), (Ib) and (IIb), enantiomers thereof and diastereomers thereof.

The invention provides the methods for regulating the activity of BTK, and treating or inhibiting diseases associated with the activity of BTK. It was confirmed that the compounds herein inhibit the activity of BTK. The present invention provides compounds of Formula (I), (II), (Ia), (IIa), (Ib) and (IIb), as pharmaceutical active ingredients for the treatment and/or prevention of the following diseases, wherein these diseases are caused by unfavorable cytokine signaling including, but not limited to:
(1) autoimmune diseases, such as chronic lymphocytic thyroiditis, hyperthyroidism, insulin-dependent diabetes mellitus, myasthenia gravis, chronic ulcerative colitis, pernicious anemia associated with chronic atrophic gastritis, Goodpasture's syndrome, pemphigus vulgaris, pemphigoid, primary biliary cirrhosis, multiple cerebrospinal sclerosis, acute idiopathic neuritis, systemic lupus erythematosus, rheumatoid arthritis, psoriasis, systemic vasculitis, scleroderma, pemphigus, mixed connective tissue disease, autoimmune hemolytic anemia, autoimmune thyroid disease, ulcerative colitis, etc.;
(2) immune disorders, such as serum sickness, asthma, allergic rhinitis, drug allergy, etc.;
(3) inflammatory diseases, such as keratitis, rhinitis, stomatitis, mumps, pharyngitis, tonsillitis, tracheitis, bronchitis, pneumonia, myocarditis, gastritis, gastroenteritis, cholecystitis, appendicitis, etc.;
(4) cancer including, but not limited to, various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL)) and other diseases that would benefit from inhibition of BTK activity.

Other diseases that would benefit from inhibition of BTK activity, including but not limited to: brain tumors, bladder cancer, stomach cancer, ovarian cancer, pancreatic cancer, breast cancer, head and neck cancer, cervical cancer, endometrial cancer, colorectal cancer, kidney cancer, esophageal cancer, prostate cancer, thyroid cancer, bone cancer, skin cancer, colon cancer, female reproductive tract tumors, lymphomas, multiple myeloma (MM), testicular cancer, and so on.

The method herein includes administering to a patient in need of a therapeutically effective amount of a compound selected from Claim 1-2.

According to standard pharmaceutical practice, the compounds (BTK inhibitors) of the invention can be used alone in a pharmaceutical formulation or with one or more additional drugs in a pharmaceutical combination, wherein the pharmaceutical formulation comprising BTK inhibitors and the additional drugs may have the same or different administration route, and the same or different administration time. The additional drugs herein include (but are not limited to):
tyrosine kinase inhibitors (e.g., Axitinib, Dasatinib, Icotinib);
topoisomerase inhibitors (e.g., topotecan);
protein kinase C inhibitors (e.g., AEB-071);
sphingosine-1-phosphate receptor agonist (e.g., fingolimod, KRP-203);
anti-T cell immunoglobulin (e.g., AtGam);
anti-IL-2 receptor antibody (e.g., daclizumab);
amides (CTX), ifosfamide (IFO), adriamycin (ADM), daunorubicin (DNR), vincristine (VCR), vinblastine (VBL), etoposide (VP16), vermeer (Vumon), carboplatin (CBP) and methotrexate (MTX) cyclosporin A, tacrolimus, sirolimus, everolimus, azathioprine, brequinar, leflunomide, LEA-29Y, anti-CD3 antibody (e.g., 0KT3), aspirin;
B7-CD28 blocking molecules (e.g., belatacept, abatacept);
CD40-CD154 blocking molecules (anti-CD40 antibodies);
Acetaminophen, ibuprofen, naproxen, piroxicam, and anti-inflammatory steroids (e.g., prednisolone or dexamethasone);
Bruton's tyrosine kinase (BTK) inhibitors include all the known, but not limited to: Ibrutinib, Compound **3,** Compound **5,** ACP-196 (Acerta Pharma), BGB-3111 (BeiGene), AVL-292 (Celgene), ONO-4059 (One Pharmaceutical), HM71224 (Hanmi Pharmaceutical), RN486 (Roche), CNX-774, CGI-11746 and so on.

mTOR inhibitors include all the known, but not limited to: everolimus, rapamycin, XL388, GDC-0349, AZD2014, AZD8055, GSK105965, MLN0128, Ridaforlimus and so on.

Immunomodulatory drugs (IMiDs) include all the known, but not limited to: Thalidomide, Revlimid, Pomalidomide, CC-122, and CC-220.

PI3K kinase inhibitors include all the known, but not limited to: BTG226, PF-05212384 (Gedatolisib), GDC-0980 (Apitolisib), GSK2126458, BEZ235, IPI-145 (Duvelisib), CAL-101 (Idelalisib) and so on.

Bcl-2 inhibitors include all the known, but not limited to: ABT-199 (Venetoclax), BI-97C1 (sabutoclax) and so on.

TOPK inhibitors include all the known, but not limited to: OTS964 (Oncotherapy Science).

JAK3 kinase inhibitors include all the known, but not limited to: Tofactinib.

JAK1/2 kinase inhibitors include all the known, but not limited to: Ruxolitinib.

ALK kinase inhibitors include all the known, but not limited to: Crizotinib, Ceritinib, and CH5424802 (Alectinib).

The present invention also relates to optimizing and screening various specific pharmaceutical compositions *in vitro* and *in vivo* animal tumor models. The compound or pharmaceutical composition, which could kill tumor cells, is not necessarily effective in animal tumor models, because of the drug pharmacokinetic problems. Therefore, without animal tumor inhibition data, *in vitro* inhibition data alone cannot demonstrate the druggability of a compound or a pharmaceutical composition. The present invention takes the oral gavage administration (rather than intraperitoneal injection or intravenous injection) to avoid the uncertainties of pharmacokinetics, optimize and screen various compounds and pharmaceutical compositions in a variety of animal tumor models to achieve druggability assessment.

Existing technology claims that ALK inhibitor and BTK inhibitors have synergistic effect on inhibition of tumor cells *in vitro,* while the present invention shows the opposite result: ALK inhibitor (Ceritinib) and BTK inhibitor Ibrutinib have no synergistic effect.

Existing technology also claims that JAK2 inhibitor and BTK inhibitors have synergistic effect on inhibition of tumor cells *in vitro,* while the present invention shows the opposite result: JAK1/2 inhibitor (Ruxolitinib) and BTK inhibitors, or mTOR inhibitors, or immunomodulatory drugs not only have no synergistic effect, but also do not have any inhibition of TMD-8 tumor cells.

The present invention discloses "two in one" and "three in one" pharmaceutical combinations (pharmaceutical compositions or pharmaceutical combinations), which are superior to single targeted drugs. "Two in one" and "three in one" pharmaceutical combinations have synergistic effects at lower doses. The formulations of the pharmaceutical combination in the present invention are not limited to the specific ratio disclosed herein for *in vitro* and *in vivo* assay.

The invention also relates to the optimization of the pharmaceutical combination based on multiple key signaling pathways, which control cell growth, proliferation, survival, and apoptosis. Single targeted therapy often brings drug resistance caused by gene mutations, which results in reduced drug efficacy and leads to disease recurrence. The pharmaceutical combinations can overcome drug resistance and achieve better therapeutic effect or cure disease. The present invention shows that pharmaceutical combinations have a synergistic effect and synthetic lethal capacity on tumor cells. The activity of pharmaceutical combinations is 100 times higher than that of single targeted drugs. Compared with single targeted drugs, pharmaceutical combinations also show remarkable effect in various xenograft models by oral gavage administration, at much lower doses (for example: 1/18 Compound **3** (BTK inhibitor) + 1/6 Everolimus (mTOR inhibitor) + 1/6 Pomalidomide (IMiD)) and possess far better therapeutic effects than single targeted drugs. The "two in one" pharmaceutical combinations result in complete tumor regression in a 15-day treatment cycle, while the "three in one" pharmaceutical combinations result in complete tumor regression in a shorter treatment cycle (9 days). Furthermore, the tumor did not rebound within 12 days after stopping the administration, which was significantly better than single targeted therapy. Single targeted therapy not only requires long-term treatment, but is also often accompanied by tumor rebound, drug resistance, and cancer recurrence. The optimized pharmaceutical combinations have better security and a broader therapeutic window than single targeted drugs, because the doses of the combinations are much lower than each single drug and at the same time, the combinations show more remarkable effect. The unique properties of the above pharmaceutical combinations in the present invention may bring new hope to patients with refractory cancer.

In the present invention, the "three in one" pharmaceutical combinations where the BTK inhibitor concentration was as low as 10 nM inhibited as much as 95% tumor cell viability. This was unexpected and cannot be inferred or guided by the existing invention. The inhibition of cell viability was tested after incubation of 48 hours. If the incubation time increased to 72-96 h, pharmaceutical compositions would display much stronger inhibition of cell viability. The present invention showed that the inhibition of cell viability *in vitro* was correlative to that of the tumor *in vivo.* Inhibition of cell viability *in vitro* can predict tumor inhibition *in vivo.* The efficient inhibition of cell viability *in vitro* may lead to tumor regression or complete disappearance at the lowest concentration (e.g., 10 nM). The pharmaceutical compositions disclosed herein could inhibit and kill tumor cells efficiently at concentrations as low as 10 nM, which ensures the invention has very significant clinical applications. In the treatment of patients, drug concentrations of the combination in the tumor cells could easily reach 10-100 nM, and thus resulting in a better therapeutic effect. At present, single targeted therapy or two-pathway combination therapy is effective in inhibiting the growth of tumor cells at the compound concentration of 1000 nM. For example, ABT-199 inhibited TMD-8 cell viability at 1000 nM, 100 nM, and 10 nM by 37.6%, 18.8%, and 11.1%, respectively. The "two in one" compositions of ABT-199 and the BTK inhibitor (Compound 3) inhibited TMD-8 cell viability by 85.97%, 79.99%, and 65.36%, respectively. The "three in one" compositions of ABT-199, BTK inhibitor (Compound **3**), and PI3K inhibitor inhibited TMD-8 cell viability by 95.56%, 95.30%, and 94.62%, respectively. "Three in one" pharmaceutical composition, which comprises ABT-199, Compound **3** (a BTK inhibitor), and mTOR inhibitor everolimus also efficiently inhibited TMD-8 cell viability by 93.44%, 94.73%, and 94.65%, respectively. The super synergistic effect in the present invention is not inferred or guided by the existing technology.

The present invention provides (1) the "three in one" combination of Bruton's tyrosine kinase (BTK) inhibitors, mTOR kinase inhibitors, and immunomodulatory drugs (IMiDs) is one of the best combinations; the other two best "three in one" combinations are: (2) Bruton's tyrosine kinase (BTK) inhibitors, mTOR kinase inhibitors, and Bcl-2 inhibitors; (3) Bruton's tyrosine kinase (BTK) inhibitors, PI3K inhibitors, and Bcl-2 inhibitors. The second best pharmaceutical combinations are "Two in one" pharmaceutical combination comprising Bruton's tyrosine kinase (BTK) inhibitors and mTOR kinase inhibitor, "Two in one" pharmaceutical combination comprising Bruton's tyrosine kinase (BTK) inhibitors and pomalidomide (IMiDs), "Two in one" pharmaceutical combination comprising Bruton's tyrosine kinase (BTK) inhibitors and TOPK inhibitors, "Two in one" pharmaceutical combination comprising TOPK inhibitors and PI3K inhibitors, and "Two in one" pharmaceutical combination comprising Bruton's tyrosine kinase (BTK) inhibitors and PI3K inhibitors.

The present invention shows that some combinations have a synergistic inhibition effect, and some have no synergistic effect and may be counterproductive. Signaling pathways are excessive, intersect, and are complex. The combinations, which can simultaneously inhibit multi-signal pathway, are much more complex. Therefore, the super synergistic effects of the three best "three in one" combinations of this invention are not inferred or guided by the existing technology.

The present invention provides a method for the optimization of cell viability inhibition *in vitro,* tumor inhibition *in vivo* and a variety of tumor models, and solves the evaluation problem of the druggability of pharmaceutical combinations.

The present invention shows that, in animal tumor models by oral gavage administration, only "three in one" pharmaceutical compositions (Bruton's tyrosine kinase (BTK) inhibitor, mTOR kinase inhibitor, and immunomodulatory drugs (IMiD)) and "two in one" (Bruton's tyrosine kinase (BTK) inhibitor and mTOR kinase inhibitors) can lead to complete tumor disappearance, and after stopping the administration, tumor rebound was not observed. Other combinations only inhibit tumor growth, and must be administered continuously to inhibit tumor growth. Everolimus monotherapy could result in tumors completely disappearing in high doses, but tumors quickly rebounded after administration, which is a defect of single targeted therapy. The present invention solves this defect, and it is expected to cure cancer in individual cases.

The invention not only verifies the outstanding inhibition effect of pharmaceutical combinations in the sensitive TMD-8 tumor model, but also proves the good inhibition effect in insensitive DoHH2 tumor models and in resistant and refractory WSU-DLCL tumor models, respectively. The efficacy of the "three in one" pharmaceutical combinations is still the best, although tumors did not disappear completely in the last two models. For example, compound EZ-6438 (Histone Methyltransferase EZH2 inhibitor) can achieve a similar effect as the WSU-DLCL tumor model by gavage to mice at very high doses (480 mg/kg/day) according to Epizyme's report, while the "three in one" combination in the invention is only 21 mg/kg/day.

The present invention for the first time demonstrates multi-targeted combination therapy has a better effect, and greatly reduces the dose of a single drug. Therefore, it can further reduce the side effects of each drug, and make the combined treatment much safer and more effective.

Multi-targeted combination therapy of the present invention can be used to treat malignant lymphoma and leukemia caused by B-cell, as well as cancer and solid tumors caused by T-cells.

Multi-targeted combination of the present invention can also be used to treat rheumatoid arthritis and other autoimmune diseases.

The present invention also provides pharmaceutical compositions comprising Bruton's tyrosine kinase (BTK) inhibitors, mTOR inhibitors, and immunomodulator drugs (IMiDs) as active ingredients.

The present invention also provides pharmaceutical compositions comprising Bruton's tyrosine kinase (BTK) inhibitors, mTOR inhibitors, and Bcl-2 inhibitors as active ingredients.

The present invention also provides pharmaceutical compositions comprising Bruton's tyrosine kinase (BTK) inhibitors, PI3K inhibitors, and Bcl-2 inhibitors as active ingredients.

The present invention also provides the use of compounds of formulas (I), (II), (Ia), (Ib), and (IIb) for the preparation of drugs that regulate BTK activity, as well as treat or inhibit the disease associated with BTK activity.

The present invention also provides the use of compounds of formulas (I), (II), (Ia), (Ib), and (IIb), mTOR kinase inhibitors and/or immunomodulator drugs (IMiDs) for the preparation of drugs that regulate BTK activity, as well as treat or inhibit the disease associated with BTK activity.

The present invention also provides the use of compositions comprising two or three compounds selected from Bruton's tyrosine kinase (BTK) inhibitors, mTOR kinase inhibitors and immunomodulator drugs (IMiDs) for the preparation of drugs that regulate BTK activity, treat or inhibit the disease associated with BTK activity.

The present invention also provides the use of compositions comprising Bruton's tyrosine kinase (BTK) inhibitors, mTOR kinase inhibitors, and Bcl-2 inhibitors for the preparation of drugs that regulate BTK activity, treat or inhibit the disease associated with BTK activity.

The present invention also provides the use of compositions comprising Bruton's tyrosine kinase (BTK) inhibitors, PI3K kinase inhibitors, and Bcl-2 inhibitors for the preparation of drugs that regulate BTK activity, treat or inhibit the disease associated with BTK activity.

Carriers, excipients, and other additives commonly used for pharmaceutical preparations may be used to prepare pharmaceutical compositions containing one or two or more compounds of formulas (I), (II), (Ia), (Ib), and (IIb) or pharmaceutically acceptable salts thereof as active ingredients.

The administration forms may be oral dosage forms, such as tablets, pills, capsules, granules, powders, emulsions, syrups, suspensions, liquid preparations, or non-oral dosage forms, such as intravenous injection or intramuscular injection, suppository, subcutaneous agent, transdermal agent, nasal agent, inhalation. Symptoms, age, sex, etc. of the individual patient should be considered in order to properly determine the dose of a compound. Generally speaking, in the case of oral administration, daily doses for adult patients of the compound is about 0.001 mg/kg to 100 mg/kg, a single dose or divided into 2 to 4 times daily. In the case of intravenous administration according to the patient symptoms, generally speaking, daily doses for adult patients are 0.0001 mg/kg to 10 mg/kg, once to more times daily. Further, in the case of using the inhalant administration, generally speaking, daily doses for adult patients are 0.0001 mg/kg to 1 mg/kg, one or more times daily.

In the present invention, solid compositions for oral administration may be tablets, powders, granules, and the like. In such solid compositions, one or more active substance with at least one inert excipient (e.g., lactose, mannitol, glucose, hydroxypropylcellulose, microcrystalline cellulose, starch, poly vinyl pyrrolidone, magnesium aluminum silicate, etc.) were mixed. According to a conventional method, the composition may contain inert additives such as lubricants (e.g., magnesium stearate), disintegrating agents (e.g., sodium carboxymethyl starch) and dissolution aids. If necessary, tablets or pills may be coated with sugar coating or a gastric or enteric coating agent.

The liquid compositions for oral administration include pharmaceutically acceptable emulsions, solutions, suspensions, syrups, elixirs, and commonly used inert diluent (e.g., purified water, ethanol). In addition to the inert diluent, the composition may also contain additives such as solubilizing agents, wetting agents, suspending agents, and sweeteners, flavoring agents, flavoring agents, and preservatives.

Injections for parenteral administration include sterile aqueous or non-aqueous liquid preparations, suspensions, and emulsions. Diluent aqueous solution can be used (for example) include distilled water for injection and physiological saline. Non-aqueous diluent solution can be used (e.g.) include propylene glycol, polyethylene glycol, vegetable oils (such as olive oil), alcohols (e.g., ethanol), and polysorbate 80. Such compositions may further contain isotonic agents, such as preservatives, wetting agents, emulsifying agents, dispersing agents, stabilizing agents, dissolving aids, and such additives. It may be employed by filtration through a bacteria retaining filter, adding bactericides or irradiation with light to a method of sterilizing the composition. In addition, these compositions may be made into sterile solid compositions before use and then sterile water or a sterile solvent added for injection prior to use as a dissolved or suspended liquid.

Transmucosal agents such as inhalations and nasal agents and the like, can be solid, liquid, or semi-solid state of use, and can be in accordance with conventionally known methods used to prepare these transmucosal agents. For example, an excipient may be added as needed (e.g., lactose and starch), pH adjusting agent, a preservative IJ, surfactants, lubricants IJ, stabilizing, and thickening agents and the like. A suitable inhalation or insufflation device can be used for administration. For example, metered dose inhaler devices may be used with a known device or sprayer, the compound can be used alone or as a mixture after the powder formulation is administered. In addition, after the compound may be combined with a pharmaceutically acceptable carrier, it can be administered as a solution or suspension. The dry powder inhaler or the like may be used for a single dose or multiple doses, and can use a dry powder or a powder-containing capsule. Further, a pressurized aerosol spray can also be used in the form to be administered by the use of a suitable propellant (e.g., chlorofluoroalkane, hydrofluoroalkane, or a suitable gas such as carbon dioxide).

**Table 1. Representative compounds for BTK Inhibitors**

| Compoun d | Structure | Name | M+1 |
|---|---|---|---|
| **1** | 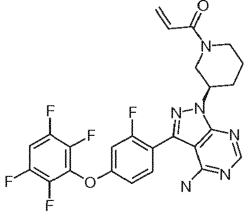 | 1-[(R)-3-[4-amino-3-[2-fluoro-4-(2,3,5,6-trifluorophenoxy)phenyl]-1H-pyrazolo[3,4-d]pyrimidin-1-yl]piperidin-1-yl]prop-2-en-1-one | 531 |
| **2** | 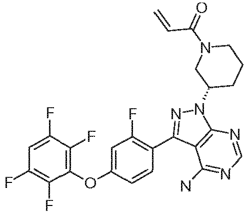 | 1-[(S)-3-[4-amino-3-[2-fluoro-4-(2,3,5,6-trifluorophenoxy)phenyl]-1H-pyrazolo[3,4-d]pyrimidin-1-yl]piperidin-1-yl]prop-2-en-1-one | 531 |
| **3** | 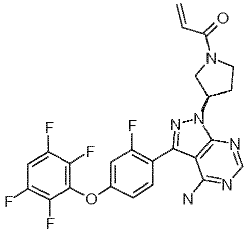 | 1-[(R)-3-[4-amino-3-[2-fluoro-4-(2,3,5 ,6-tetrafluorophenoxy)phenyl] -1H-pyrazolo [3,4-d]pyrimidin-1-yl]pyrrolidin-1-yl]prop-2-en-1-one | 517 |
| **4** | 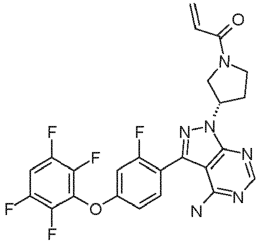 | 1-[(S)-3-[4-amino-3-[2-fluoro-4-(2,3,5,6-tetrafluorophenoxy)phenyl] -1H-pyrazolo[3,4-d]pyrimidin-1-yl]pyrrolidin-1-yl]prop-2-en-1-one | 517 |
| **5** | 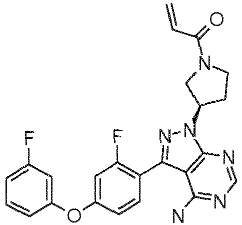 | 1-[(R)-3-[4-amino-3-[2-fluoro-4-(3-fluorophenoxy)phenyl]-1H-pyrazolo[3,4-d]pyrimidin-1-yl]pyrrolidin-1-yl]prop-2-en-1-one | 463 |
| **6** | 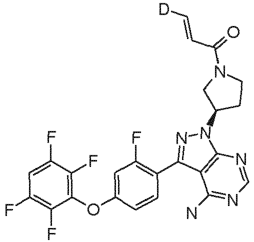 | (*E*)-1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]-3-deuterium-prop-2-en-1-one | 518 |
| **7** | 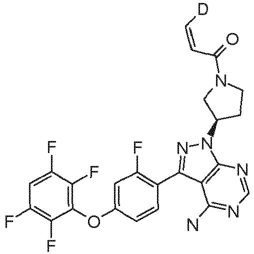 | (Z)-1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]-3-deuterium-prop-2-en-1-one | 518 |
| **7x** | 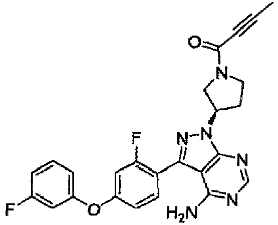 | 1-[(R)-3-[4-amino-3-[2-fluoro-4-(3-fluorophenoxy)phenyl]-1H-pyrazolo[3,4-d]pyrimidin-1-yl]pyrrolidin-1-yl]-butyl-2-yn-1-one | 475 |

| | | | |
|---|---|---|---|
| **Note:** If there are differences between the structure and name, the structure will prevail. | | | |

### BRIEF DESCRIPTION OF THE FIGURES

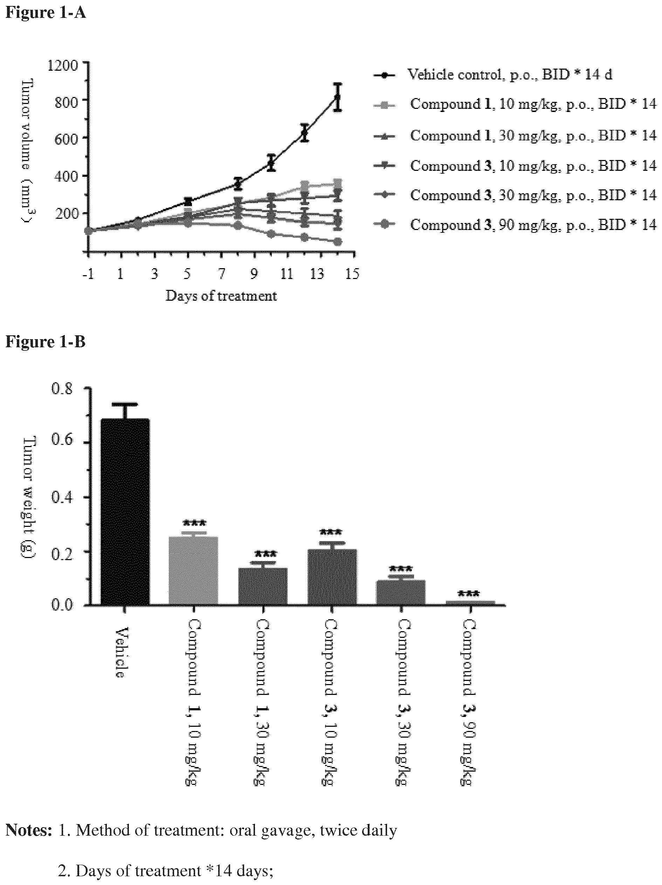

**Figure 1-A****:** Antitumor effect of multiple doses of Compound **1** and **3** on tumor volume in TMD-8 lymphoma xenograft model in SCID mice
**Figure 1-B****:** Antitumor effect of Compound **1** and **3** on tumor weight in TMD-8 lymphoma xenograft model in SCID mice

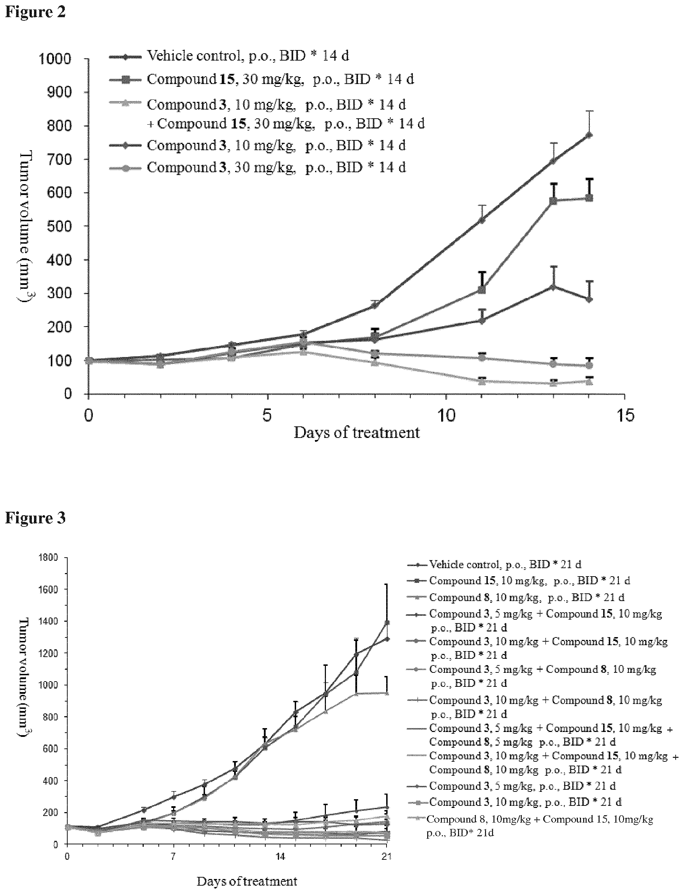

**Figure 2****:** Antitumor effect of Compound **3, 15** and their combination in TMD-8 lymphoma xenograft model in SCID mice
**Figure 3****:** Antitumor effect of Compound **3, 8, 15** and their combinations in TMD-8 lymphoma xenograft model in SCID mice

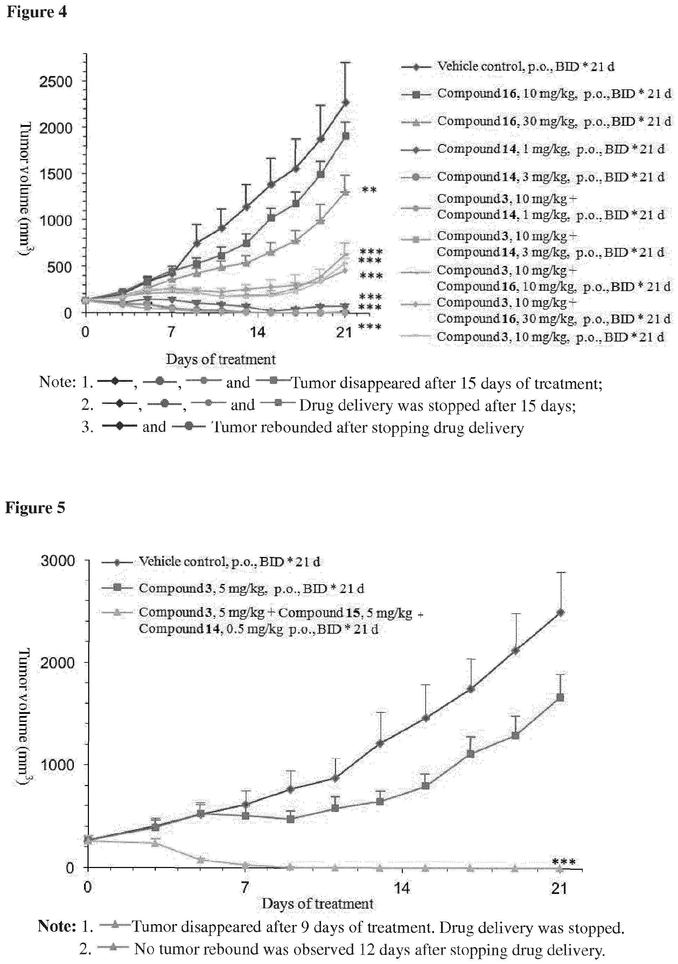

**Figure 4****:** Antitumor effect of Compound **3, 14, 16** and their combinations in TMD-8 lymphoma xenograft model in SCID mice
**Figure 5****:** Antitumor effect of Compound **3, 14, 15** and their combination in TMD-8 lymphoma xenograft model in SCID mice

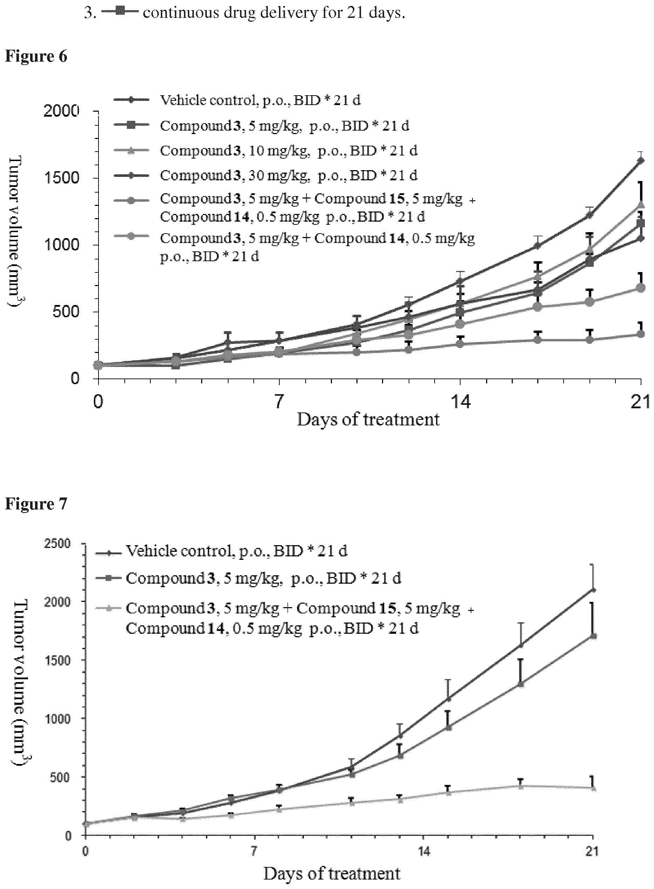

**Figure 6****:** Antitumor effect of Compound **3, 14, 15** and their combinations in DoHH-2 lymphoma xenograft model in SCID mice
**Figure 7****:** Antitumor effect of Compound **3, 14, 15** and their combination in DoHH-2 lymphoma xenograft model in SCID mice

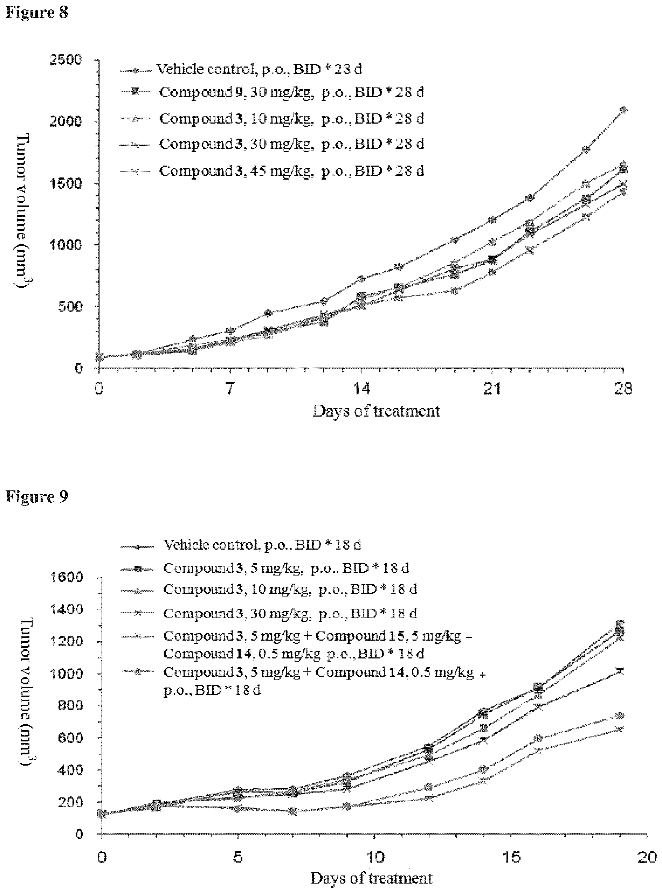

**Figure 8****:** Antitumor effect of Compound **3** and **9** in resistant ESU-DLCL2 mice model
**Figure 9****:** Antitumor effect of Compound **3, 14, 15** and their combination in resistant ESU-DLCL2 mice model.

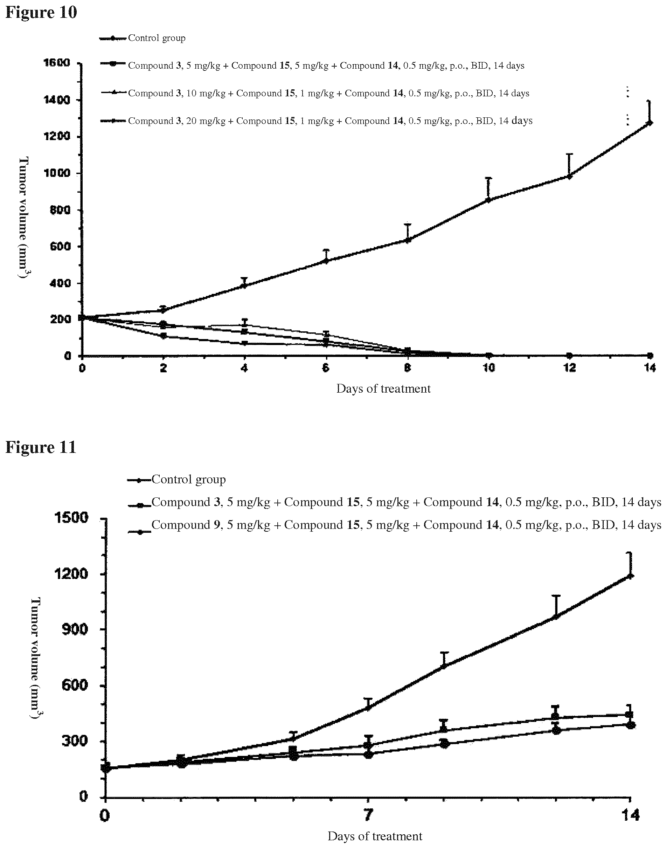

**Figure 10****:** Antitumor effect of Compound **3, 14, 15** and their "3-in-1" combination in TMD-8 lymphoma xenograft model in mice
**Figure 11****:** Antitumor effect of Compound **3, 14, 15** and Compound **9, 14, 15** and their "3-in-1" combination in DoHH-2 lymphoma xenograft model in mice

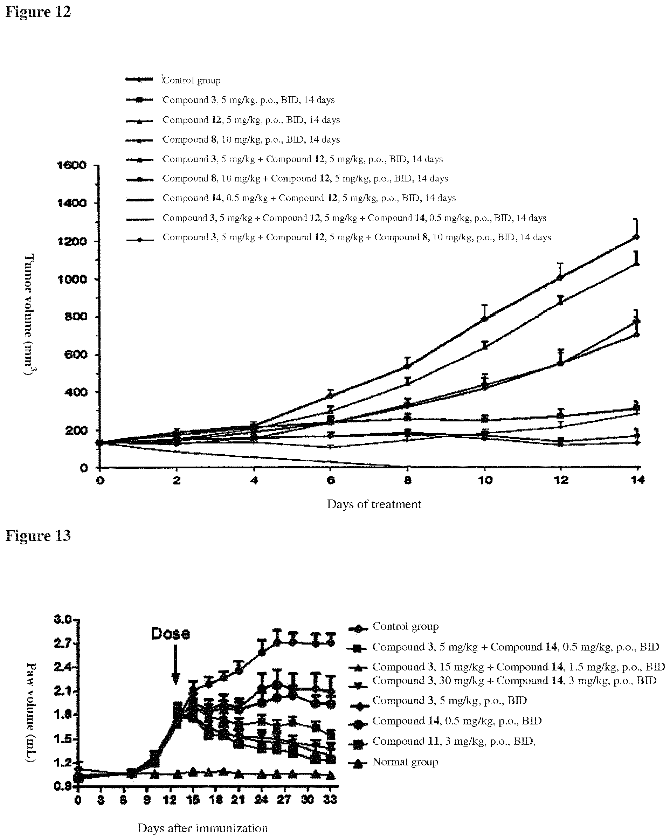

**Figure 12****:** Antitumor effect of Compound **3, 8, 12** and their "2-in-1" and "3-in-1" combinations in TMD-8 lymphoma xenograft model in mice
**Figure 13****:** foot volume - adjuvant-induced arthritis

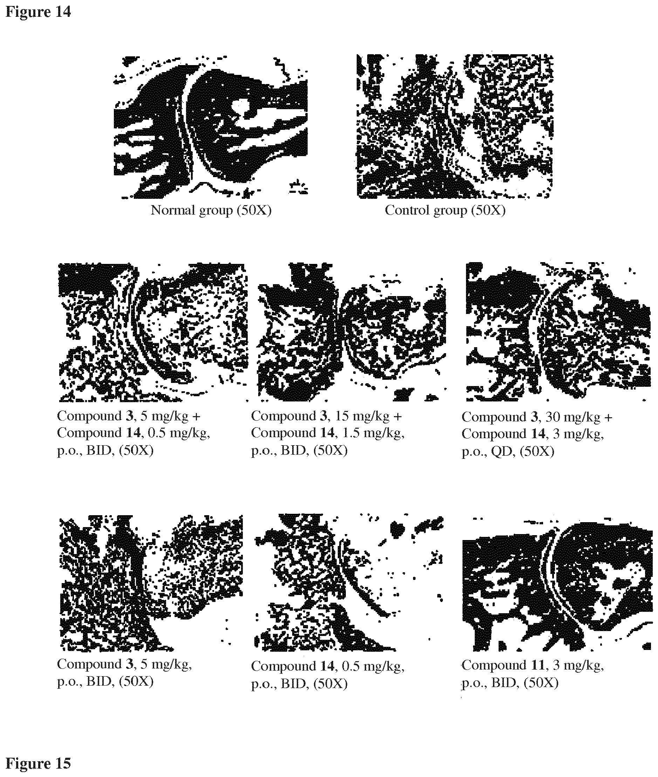

**Figure 14****:** histopathology - adjuvant-induced arthritis

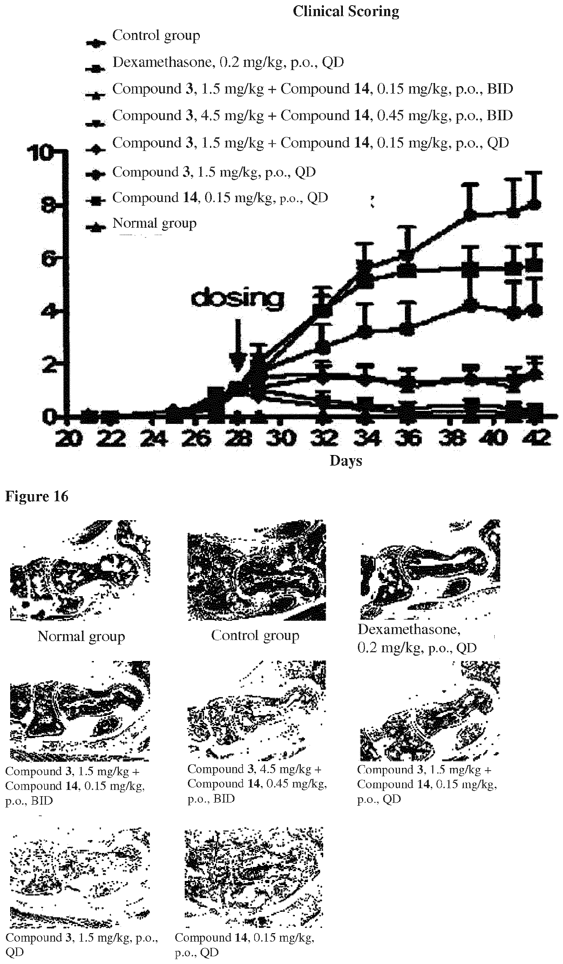

**Figure 15****:** clinical review - collagen-induced arthritis
**Figure 16****:** histopathology - collagen-induced arthritis

### Example 1

### Compound 1 and Compound 2

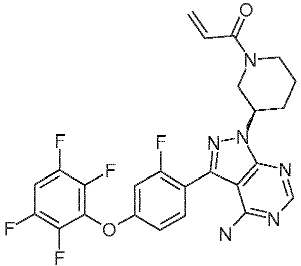

### 1-[(R)-3-[4-amino-3-[2-fluoro-4-(2,3,5,6-trifluorophenoxy)phenyl]-1H-pyrazolo[3,4-d]pyrimidin-1-yl]piperidin-1-yl]prop-2-en-1-one

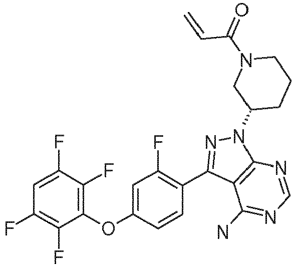

### 1-[(S)-3-[4-amino-3-[2-fluoro-4-(2,3,5,6-trifluorophenoxy)phenyl]-1H-pyrazolo[3,4-d]pyrimidin-1-yl]piperidin-1-yl]prop-2-en-1-one

### Step A:

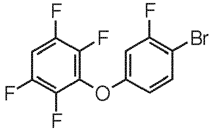

### 3-(4-bromo-3-fluorophenoxy)-1,2,4,5-tetrafluorobenzene

### Procedure:

Potassium carbonate (68.0 g, 492.1 mmol, 2.0 eq.) and the compound 1,2,3,4,5-pentafluorophenyl (49.6 g, 295.3 mmol, 1.2 eq.) was added to a solution of 3-fluoro-4-bromophenol (47.0 g, 246.1 mmol, 1.0 eq.) in DMF (500 mL). The reaction was stirred at 100 °C for 12 hours. Solvent was removed under reduced pressure. The residue was dissolved in ethyl acetate (300 mL), washed with water (100 mL) and brine (100 mL). The organic phase was dried over anhydrous sodium sulfate and concentrated to give the title compound (78 g, yield: 93%).

### Step B:

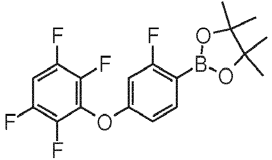

### 2-[2-fluoro-4-(2,3,5,6-tetrafluorophenoxy)phenyl]-4,4,5,5-tetramethyl-1,3,2-dioxaborolane Procedure:

3-(4-bromo-3-fluorophenoxy)-1,2,4,5-tetrafluorobenzene (73 g, 215.3 mmol, 1.0 eq.), bis pinacolato boronate (65.6 g, 258.4 mmol, 1.2 eq.), potassium acetate (31.6 g, 322.9 mmol, 1.5 eq.) and (dppf)PdCl₂ (9.4 g, 12.8 mmol, 0.06 eq.) were added to 1,4-dioxane (1 L). The resulting mixture was stirred at 80 °C for 14 hours under nitrogen. After cooling to room temperature, the reaction mixture was filtered through Celite. The filtrate was concentrated to give the crude product, which was purified by silica gel column chromatography (eluent: petroleum ether) to give the title compound (60 g, yield: 72%).

### Step C:

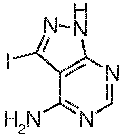

### 3-iodo-1H-pyrazolo[3,4-d]pyrimidin-4-amine

### Procedure:

NIS (250 g, 1.11 mol, 1.5 eq.) was added to a solution of 1H-pyrazolo[3,4-d]pyrimidin-4-amine (100 g, 0.74 mol, 1.0 eq.) in DMF (800 mL) . The reaction was stirred at 80∼85 °C for 16 hours under nitrogen. The reaction mixture was filtered. The filter cake was washed with ethanol (1000 mL x 3) to give the title compound (184 g, yield: 95%).

### Step D:

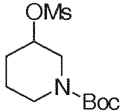

### tert-butyl 3-(methylsulfonyloxy)piperidine-1-carboxylate

### Procedure:

Triethylamine (15 g, 150 mmol, 3.0 eq.) and methanesulfonyl chloride (6.3 g, 55 mmol, 1.1 eq.) were sequentially added dropwise to a solution of 3-hydroxy-piperidine-1-carboxylate (10.0 g, 50 mmol, 1.0 eq.) in dichloromethane (100 mL) at 0 °C. The reaction was stirred at 20 °C for 1 hour, and then quenched with saturated NaHCO₃ (100 mL). The resulting mixture was extracted with dichloromethane (200 mL x 3). The combined organic phases were dried over anhydrous sodium sulfate, and concentrated under reduced pressure to give the title compound (13 g, yield: 95%).

### Step E:

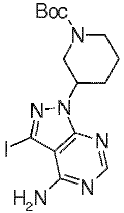

### tert-butyl 3-(4-amino-3-iodo-1H-pyrazolo[3,4-d]pyrimidin-1-yl)piperidine-1-carboxylate Procedure:

Cesium carbonate (20.2 g, 62 mmol, 2.0 eq.) and 3- (methylsulfonyloxy) piperidine-1-carboxylate (13 g, 46.5 mmol, 1.5 eq.) was added to a solution of 3-iodo-1H- pyrazolo[3,4-d]-pyrimidin-4-amine (8.1 g, 31 mmol, 1.0 eq) in DMF (50 mL) at 0 °C. The reaction was stirred at 80 °C overnight. After cooling to room temperature, the mixture was filtered through celite, and concentrated under reduced pressure to give the crude product, which was purified by silica gel column chromatography (eluent: ethyl acetate) to give the title compound (5 g, yield: 25%) .

### Step F:

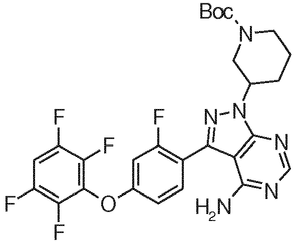

### tert-butyl 3-[4-amino-3-[2-fluoro-4-(2,3,5,6-tetrafluorophenoxy)phenyl]-1H-pyrazolo[3,4-d]pyrimidin-1-yl]piperidine-1-carboxylate

### Procedure:

Tert-butyl 3-(4-amino-3-iodo-1H-pyrazolo[3,4-d]pyrimidin-1-yl)piperidine-1-carboxylate (7.6 g, 17.1 mmol, 1.0 eq.), 2-[2-fluoro-4-(2,3,5,6-tetrafluorophenoxy)phenyl]-4,4,5,5-tetramethyl-1,3,2-dioxaborolane (8.6 g, 22.3 mmol, 1.3 eq.), potassium phosphate (7.3 g, 34.2 mmol, 2.0 eq.), and Pd-118 (0.56 g, 0.855 mmol, 0.05 eq.) were added to a mixture of 1,4-dioxane/water (5/1, v/v, 240 mL). The reaction stirred at 60 °C for 12 hours under a nitrogen atmosphere. After cooling to room temperature, the reaction mixture was poured into ice water (300 mL) and then extracted with ethyl acetate (100 mL x 4). The combined organic phases were dried over anhydrous sodium sulfate, and concentrated to give the crude product, which was purified by silica gel column chromatography separation (eluent: ethyl acetate) to give the title compound (6.8 g, yield: 69%).

### Step G:

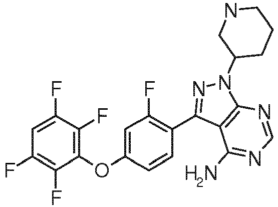

### 3-[2-fluoro-4-(2,3,5,6-tetrafluorophenoxy)phenyl]-1-(piperidin-3-yl)-1H-pyrazolo[3,4-d]pyrimidin-4-amine

### Procedure:

HCl/EA (20 mL, 4 mol/L) was added to a solution of tert-butyl 3-[4-amino-3-[2-fluoro-4-(2,3,5,6-tetrafluorophenoxy)phenyl]-1H-pyrazolo[3,4-d]pyrimidin-1-yl]piperidine-1-carboxylate (6.8 g, 11.8 mmol) in ethyl acetate (50 mL) at 0 °C. The reaction was stirred at room temperature for 1 hour, and then concentrated to give the title compound hydrochloride (5.2 g, yield: 86%).

### Step H:

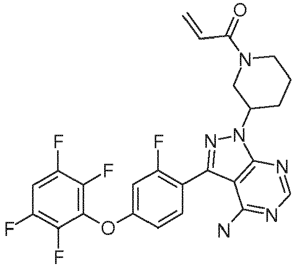

### 1-(3-(4-amino-3-(2-fluoro-4-(2,3,5,6-tetrafluorophenoxy)phenyl)-1H-pyrazolo[3,4-d]pyrimidin-1-yl)piperidin-1-yl)prop-2-en-1-one

### Procedure:

Triethylamine (887 mg, 8.7 mmol, 3.0 eq.) and acryloyl chloride (0.26 g, 2.9 mmol, 1.0 eq.) were sequentially added dropwise to a solution of 3-[2-fluoro-4-(2,3,5,6-tetrafluorophenoxy)phenyl]-1-(piperidin-3-yl)-1H-pyrazolo[3,4-d]pyrimidin-4-amine (1.5 g, 2.9 mmol, 1.0 eq.) in dichloromethane (10 mL). The reaction was stirred at 0 °C for 1 hour, quenched with water (5 mL), diluted with dichloromethane (50 mL), and washed with water (30 mL x 2) and saturated brine (30 mL). The organic phase was dried over anhydrous sodium sulfate and concentrated to give the crude product, which was purified by silica gel column chromatography to give the title compound (eluent: petroleum ether: ethyl acetate = 1:0 ∼ 1:1) (0.94 g, yield: 64%).
Spectroscopic data:
LC/MS (method: UFLC): RT = 3.130 min; m/z = 531.1 [M+H]⁺; Total running time = 7 min.
¹H NMR (400MHz, DMSO-*d₆*) δ 8.22 (s, 1H), 8.00-7.91 (m, 1H), 7.55-7.46 (m, 1H), 7.27 (dd, *J* = 2.4, 10.8 Hz, 1H), 7.12 (dd, *J* = 2.4, 8.8 Hz, 1H), 6.88-6.65 (m, 1H), 6.13-6.02 (m, 1H), 5.70-5.56 (m, 1H), 4.71-4.65 (m, 1H), 4.54-4.51 (m, 0.5H), 4.20-4.17 (m, 1H), 4.07-4.04 (m, 0.5H), 3.67-3.60 (m, 0.5H), 3.17-3.12 (m, 1H), 2.98-2.94 (m, 0.5H), 2.26-2.21 (m, 1H), 2.11-2.06 (m, 1H), 1.92-1.89 (m, 1H), 1.58-1.54 (m, 1H).

### Step I:

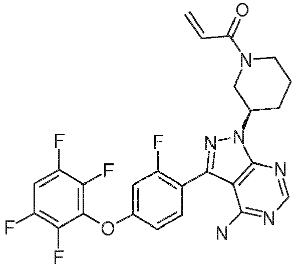

### 1-[(R)-3-[4-amino-3-[2-[fluoro-4-(2,3,5,6-tetrafluorophenoxy)phenyl]-1H-pyrazolo[3,4-d]pyrimidin-1-yl]piperidin-1-yl]prop-2-en-1-one

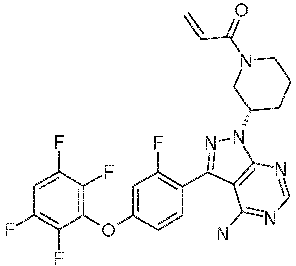

### 1-[(S)-3-[4-amino-3-[2-fluoro-4-(2,3,5,6-tetrafluorophenoxy)phenyl]-1H-pyrazolo[3,4-d]pyrimidin-1-yl]piperidin-1-yl]prop-2-en-1-one

### Procedure:

1-[3-[4-amino-3-[2-[fluoro-4-(2,3,5,6-tetrafluorophenoxy)phenyl]-1H-pyrazolo[3,4-d]pyrimidin -1-yl]piperidin-1-yl]prop-2-en-1-one (750 mg) was separated by SFC chiral resolution (CO₂ :C₂H₅OH(0.2%DEA), v/v, 200 ml/min) to give Compound **1** 1-[(R)-3-[4-amino-3-[2-fluoro-4-(2,3,5,6-tetrafluorophenoxy)phenyl]-1H-pyrazolo[3,4-d]pyrimidin-1-yl]piperidin-1-yl]prop-2-en-1 - one (280 mg, ee: 100%) and Compound **2** 1-[(S)-3-[4-amino-3-[2-fluoro-4-(2,3,5,6-tetrafluorophenoxy)phenyl]-1H-pyrazolo[3,4-d]pyrimidin-1-yl]piperidin-1-yl]prop-2-en-1-one (330 mg, ee: 98%).
Spectroscopic data:

### Compound 1:

LC/MS (method: UFLC): RT = 3.002 min; m/z = 531.1 [M+H]⁺; Total running time = 7 min.
¹H NMR (400MHz, CDCl₃) δ 8.36 (s, 1H), 7.58 (t, *J* = 8.4 Hz, 1H), 7.09-7.04 (m, 1H), 6.94-6.88 (m, 2H), 6.62-6.54 (m, 1H), 6.32-6.25 (m, 1H), 5.73-5.63 (m, 1H), 5.56-5.51 (m, 1H), 4.90-4.85 (m, 1.5H), 4.59-4.56 (m, 0.5H), 4.21-4.17 (m, 0.5H), 4.04-4.01 (m, 0.5H), 3.76-3.71 (m, 0.5H), 3.40-3.35 (m, 0.5H), 3.22-3.15 (m, 0.5H), 2.93-2.87 (m, 0.5H), 2.39-2.27 (m, 2H), 2.04-1.68 (m, 2H).

### Compound 2:

LC/MS (method: UFLC): RT = 3.006 min; m/z = 531.1 [M+H]⁺; Total running time = 7 min.
¹H NMR (400MHz, CD₃OD) δ 8.24 (s, 1H), 7.62 (t, J = 8.4 Hz, 1H), 7.50-7.45 (m, 1H), 7.09-7.01 (m, 2H), 6.85-6.63 (m, 1H), 6.21-6.09 (m, 1H), 5.77-5.61 (m, 1H), 4.63-4.59 (m, 1H), 4.23-4.07 (m, 1.5H), 3.90-3.85 (m, 0.5H), 3.51-3.45 (m, 0.5H), 3.34-3.17 (m, 1.5H), 2.40-2.23 (m, 2H), 2.08-2.05 (m, 1H), 1.75-1.71 (m, 1H).

### Example 2

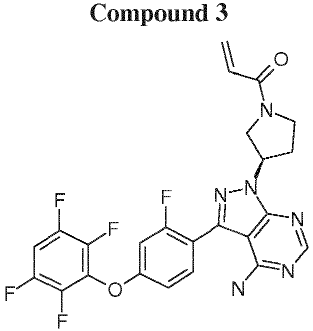

### 1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

### Method 1:

### Step A:

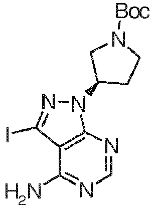

### (R)-tert-butyl 3-(4-amino-3-iodo-1H-pyrazolo[3,4-d]pyrimidin-1-yl)pyrrolidine-1-carboxylate Procedure:

DIAD (27.6 g, 137.5 mmol, 1.5 eq.) was added dropwise to a mixture of 3-iodo-1H-pyrazolo[3,4-d]pyrimidin-4-amine (24 g, 92 mmol, 1.0 eq.), (S)-tert-butyl 3-hydroxypyrrolidine-1-carboxylate (26 g, 137.5 mmol, 1.5 eq) and PPh₃ (36 g, 137.5 mmol, 1.5 eq) in tetrahydrofuran (720 mL) at 0 °C and under a nitrogen atmosphere. The reaction was stirred at 0 °C for 1 hour, then stirred overnight at room temperature. After the removal of solvent under reduced pressure, acetonitrile (200 mL) was added to residue. The mixture was stirred at room temperature for 2 hours and filtered. The filter cake was washed with acetonitrile (20 mL) and dried to give the title compound (25 g, yield: 63%) .

### Step B:

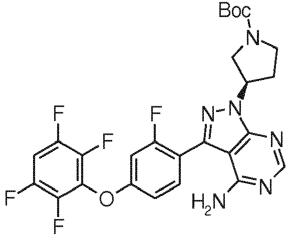

### (3R)-tert-butyl 3-[4-amino-3-[2-fluoro-4-(2,3,5,6-tetrafluorophenoxy)phenyl]-1H-pyrazolo[3,4-d]pyrimidin-1-yl]pyrrolidine-1-carboxylate

### Procedure:

(R)-tert-butyl 3-(4-amino-3-iodo-1H-pyrazolo[3,4-d]pyrimidin-1-yl)pyrrolidine-1-carboxylate (25 g, 58 mmol, 1.0 eq.), 2-[2-fluoro-4-(2,3,5,6-tetrafluorophenoxy)phenyl]-4,4,5,5-tetramethyl-1,3,2-dioxaborolane (30 g, 75.4 mmol, 1.3 eq.), potassium phosphate (25 g, 116 mmol, 2.0 eq.), and Pd-118 (750 mg, 1.16 mmol, 0.02 eq.) were added to a mixture of 1,4-dioxane/ water (5/1, v/v, 600 mL). The reaction was stirred at 60 °C overnight under a nitrogen atmosphere. After cooling to room temperature, the mixture was filtered through Celite. Filtrate was concentrated under reduced pressure. water (300 mL) was added to the residue, then extracted with ethyl acetate (300 mL x 3). The combined organic phases were dried over anhydrous sodium sulfate, and concentrated to give the title compound (60 g, crude).

### Step C:

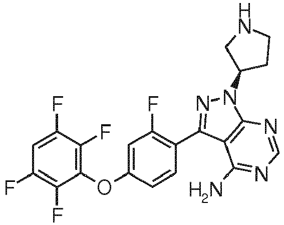

### 3-[2-fluoro-4-(2,3,5,6-tetrafluorophenoxy)phenyl]-1-((R)-pyrrolidin-3-yl)-1H-pyrazolo[3,4-d]pyrimidin-4-amine

### Procedure:

HCl/EA (100 mL, 4 mol/L) was added to a solution of (3R)-tert-butyl 3-[4-amino-3-[2-fluoro-4-(2,3,5,6-tetrafluorophenoxy)phenyl]-1H-pyrazolo[3,4-d]pyrimidin-1-yl]pyrrolidine-1-carboxylate (60 g, crude) in ethyl acetate (100 mL) at 0 °C . The reaction was stirred at room temperature for 1 hour and concentrated to dryness to give the hydrochloride salt of the title compound. Water (500 mL) was added to the reaction flask, extracted with ethyl acetate (300 mL x 3). The aqueous phase was adjusted to pH = 9, and then extracted with ethyl acetate (300 mL x 3). The combined organic phases were dried over anhydrous sodium sulfate, and concentrated under reduced pressure to give the title compound (24 g, two steps yield: 90%).

### Step D:

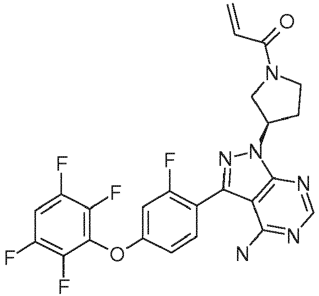

### 1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

### Procedure:

NaOH (10%, 94 mL) was added to a solution of 3-[2-fluoro-4-(2,3,5,6-tetrafluorophenoxy)phenyl]-1-[(R)-pyrrolidin-3-yl]-1H-pyrazolo[3,4-d]pyrimidin-4-amine (23.5 g, 50.75 mmol, 1.0 eq) in tetrahydrofuran (470 mL) at -5 °C, and then acryloyl chloride (5.97 g, 66 mmol, 1.3 eq.) was added dropwise. The reaction was stirred at -5 °C for 1 hour, quenched with saturated brine (100 mL), and extracted with ethyl acetate (200 mL x 3). The combined organic phases were dried over anhydrous sodium sulfate, and concentrated to give the crude product, which was purified by silica gel column chromatography (eluent: petroleum ether: ethyl acetate = 1: 3 ∼ 1: 1). The product obtained was dissolved in methanol (500 mL) and filtered. Water (1500 mL) was added to the stirred filtrate, stirred for 2 hours and filtered. The filter cake was dried under reduced pressure to give the title compound (16.5 g, yield: 63%).
Spectroscopic data:
LC/MS (method: UFLC): RT = 3.764 min; m/z = 517.0 [M+H]⁺; Total running time = 7 min.
¹H NMR (400MHz, CD₃OD) δ 8.45 (s, 1H), 7.70 (t, *J* = 8.4 Hz, 1H), 7.55-7.46 (m, 1H), 7.12-7.05 (m, 2H), 6.70-6.55 (m, 1H), 6.33-6.26 (m, 1H), 5.81-5.75 (m, 1H), 4.23-3.83 (m, 5H), 2.68-2.55 (m, 2H).

### Method 2:

### Procedure:

NaOH (216 mg, 5.40 mmol, 2.5 eq.) was added to a solution of 3-[2-fluoro-4-(2,3,5,6-tetrafluorophenoxy)phenyl]-1-[(R)-pyrrolidin-3-yl]-1H-pyrazolo[3,4-d]pyrimidin-4-amine (1.0 g, 2.16 mmol, 1.0 eq.) in tetrahydrofuran (50 mL) and water (10 mL) at 0 °C, and then a solution of chloropropionyl chloride (288 mg, 2.27 mmol, 1.05 eq.) in tetrahydrofuran (10 mL) was added dropwise. The reaction was stirred at 0 °C for 1 hour, then at 60 °C for 12 hours. After cooling to room temperature, saturated brine (10 mL) was added, and then extracted with ethyl acetate (50 mL x 3). The combined organic phases were dried over anhydrous sodium sulfate, and concentrated to give the crude product, which was purified by silica gel column chromatography (eluent: petroleum ether: ethyl acetate = 1: 3 ∼ 1: 1) to give Compound 3 (0.8 g, yield: 71%).

### Method 3:

### Procedure:

(R)-[3-(4-amino-3-iodo-1H-pyrazolo[3,4-d]pyrimidin-1-yl)pyrrolidin-1-yl]prop-2-en-1-one (100 g, 0.26 mmol, 1.0 eq.), 2-[2-fluoro-4-(2,3,5,6-tetrafluorophenoxy)phenyl]-4,4,5,5-tetramethyl-1,3,2-dioxaborolane (120 mg, 0.31 mmol, 1.2 eq.), sodium carbonate (55 mg, 0.52 mmol, 2.0 eq.) and Pd (PPh₃)₄ (30 mg, 0.026 mmol, 0.01 eq) was added to a mixture of 1,4-dioxane/water (5 mL, 1/1, v/v). The reaction was stirred under microwave irradiation at 80 °C for 30 minutes. After cooling to room temperature, the reaction mixture was filtered through Celite. The filtrate was concentrated to give the crude product, which was purified by HPLC separation on a C18 column (mobile phase: acetonitrile/water/0.5% HCl, eluent gradient 10% to 100% (volume ratio)). After the removal of volatile solvent, the desired fraction was lyophilized to give the title compound (38 mg, yield: 28%).

### Method 4:

### Compound 3 and Compound 4

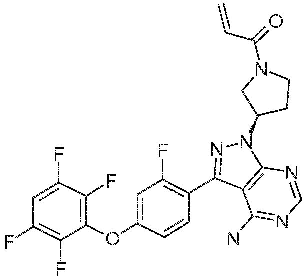

### 1-[(R)-3-[4-amino-3-[2-[fluoro-4-(2,3,5,6-tetrafluorophenoxy)phenyl]-1H-pyrazolo[3,4-d]pyrimidin-1-yl]pyrrolidin-1-yl]prop-2-en-1-one

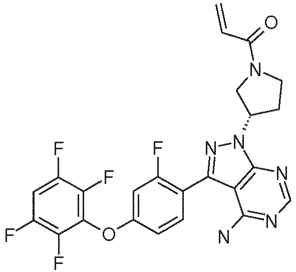

### 1-[(S)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

### Step A:

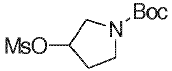

### tert-butyl 3-(methylsulfonyloxy)pyrrolidine-1-carboxylate

### Procedure:

Triethylamine (35 g, 346 mmol, 2.1 eq.) was added to a solution of 3-hydroxy-pyrrolidine-1-carboxylate (30.0 g, 163 mmol, 1.0 eq.) in dichloromethane (200 mL) at 0 °C, and then methyl chloride (36.6 g, 321 mmol, 1.9 eq.) was added dropwise. The reaction was stirred at 0 °C for 3 hours, quenched with water (20 mL), washed with water (100 mL x 2) and saturated brine (100 mL). The organic phase was dried over anhydrous sodium sulfate and concentrated to give the title compound (45.6 g, yield: 100%).

### Step B:

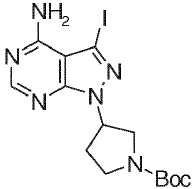

### tert-butyl 3-(4-amino-3-iodo-1H-pyrazolo[3,4-d]pyrimidin-1-yl)pyrrolidine-1-carboxylate

### Procedure:

Cesium carbonate (37 g, 115 mmol, 3.0 eq.) and the compound 3-iodo-1H-pyrazolo[3,4-d]pyrimidin-4-amine (10 g, 38 mmol, 1.0 eq.) were added to a solution of tert-butyl 3-(methylsulfonyloxy)pyrrolidine-1-carboxylate (35 g, 134 mmol, 3.5 eq.) in DMF (300 mL). The reaction was stirred at 85 °C for 12 h. After cooling to room temperature, the mixture was filtered. The filtrate was concentrated to give the crude product, which was purified by silica gel column chromatography (eluent: petroleum ether: ethyl acetate = 1:1) to give the title compound (7.0 g, yield: 44%).

### Step C:

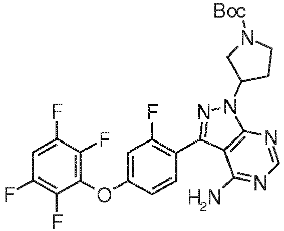

### tert-butyl 3-[4-amino-3-[2-fluoro-4-(2,3,5,6-tetrafluorophenoxy)phenyl]-1H-pyrazolo[3,4-d]pyrimidin-1-yl]pyrrolidine-1-carboxylate

### Procedure:

Tert-butyl 3-(4-amino-3-iodo-1H-pyrazolo[3,4-d]pyrimidin-1-yl)pyrrolidine-1-carboxylate (8 g, 18 mmol, 1.0 eq.), 2-[2-fluoro-4-(2,3,5,6-tetrafluorophenoxy)phenyl]-4,4,5,5-tetramethyl-1,3,2-dioxaborolane (10.7 g, 27 mmol, 1.5 eq.), potassium phosphate (7.6 g, 36 mmol, 2.0 eq.) and Pd-118 (1.2 g, 1.8 mmol, 0.1 eq.) were added to a mixture of 1,4-dioxane/water (180 mL, 5/1, v/v). The reaction was placed under nitrogen and stirred at 60 °C for 14 hours. After cooling to room temperature, the reaction mixture was poured into ice water (50 mL) and extracted with ethyl acetate (100 mL x 3). The combined organic phases were dried over anhydrous sodium sulfate and concentrated to give the crude product, which was purified by silica gel column chromatography (eluent: ethyl acetate: petroleum ether = 1: 1) to give the title compound (2.5 g, yield : 25%).

### Step D:

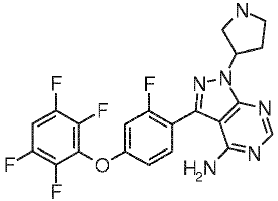

### 3-[2-fluoro-4-(2,3,5,6-tetrafluorophenoxy)phenyl]-1-(pyrrolidin-3-yl)-1H-pyrazolo[3,4-d]pyrimidin-4-amine

### Procedure:

HCl/EA (20 mL, 4 mol/L) was added to a solution of tert-butyl 3-[2-amino-3-[2-fluoro-4-(2,3,5,6-tetrafluorophenoxy)phenyl]-1H-pyrazolo[3,4-d]pyrimidin-1-yl]pyrrolidine-1-carboxylate (2.5 g, 4.4 mmol) in dichloromethane (20 mL) at 0 °C. The reaction was stirred for 1 hour at room temperature, and then concentrated under pressure to give the title compound hydrochloride (2.2 g, yield: 100%).

### Step E:

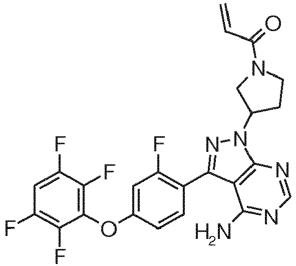

### 1-[3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

### Procedure:

Triethylamine (1.4 g, 12.8 mmol, 3.0 eq.) was added to a solution of 3-[2-fluoro-4-(2,3,5,6-tetrafluorophenoxy)phenyl]-1-(pyrrolidin-3-yl)-1H-pyrazolo[3,4-d]pyrimidin-4-amine (2.2 g, 4.4 mmol, 1.0 eq.) in dichloromethane (50 mL), then acryloyl chloride (0.38 g, 4.2 mmol, 0.95 eq.) was added dropwise at 0 °C. The reaction was stirred at 0 °C for 1 hour and quenched with water (30 mL). The aqueous phase was extracted with methylene chloride (30 mL x 3). The combined organic phases were dried over anhydrous sodium sulfate and concentrated to give the crude product, which was purified by silica gel column chromatography (eluent: ethyl acetate) to give the title compound (1.0 g, yield: 45%).
Spectroscopic data:
LC/MS (method: UFLC): RT = 2.810 min; m/z = 517.1 [M+H]+; Total running time = 7 min.

### Step F:

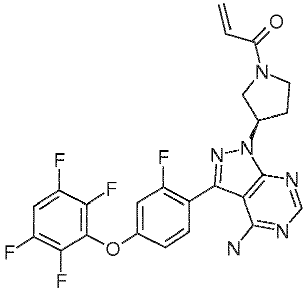

### 1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

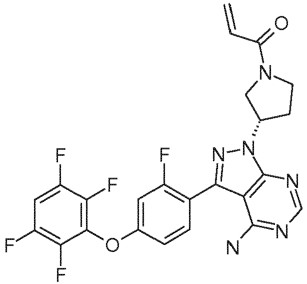

### 1-[(S)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one

### Procedure:

Racemate 1-[3-[4-amino-3-[2-fluoro-4-(2,3,5,6-tetrafluorophenoxy)phenyl]-1H-pyrazolo[3,4-d]pyrimidin-1-yl]pyrrolidin-1-yl]prop-2-en-1-one was separated by SFC chiral resolution to give Compound **3** (270 mg) and Compound **4** (320 mg).
Spectroscopic data:
LC/MS (method: UFLC): RT = 2.808 min; m/z = 517.1 [M+H]⁺; Total running time = 7 min..

### Example 3

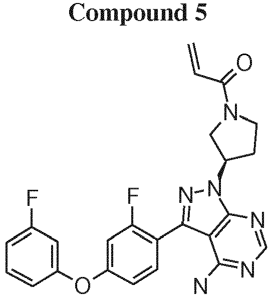

### 1-[(R)-3-[4-amino-3-[2-fluoro-4-(3-fluorophenoxy)phenyl]-1H-pyrazolo[3,4-d]pyrimidin-1-yl]pyrrolidin-1-yl]prop-2-en-1-one

### Step A:

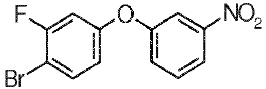

### 1-(3-fluoro-4-bromo-bromo-phenoxy)-3-benzene

### Procedure:

1-fluoro-3-nitrobenzene (29.6 g, 210 mmol, 1.0 eq.) and potassium carbonate (58 g, 420 mmol, 2.0 eq.) were added to a solution of 3-fluoro-4-bromophenol (40 g, 210 mmol, 1.0 eq.) in DMF (400 mL). The reaction was stirred at 90 °C for 12 hours under a nitrogen atmosphere. After the removal of the solvent under reduced pressure, water (300 mL) was added to the residue and then extracted with ethyl acetate (300 mL x 3). The combined organic phases were dried over anhydrous sodium sulfate and concentrated to give the title compound (65 g, yield: 100%).

### Step B:

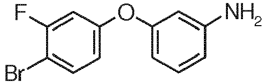

### 3-(3-fluoro-4-bromo-phenoxy)benzenamine

### Procedure:

Chloride ammonium (28 g, 525 mmol, 2.5 eq.) and iron powder (58.8 g, 1.05 mol, 5.0 eq.) were added to a solution of 1-bromo-2-fluoro-4-(3-nitrophenoxy)benzene (65 g, 210 mmol, 1.0 eq.) in ethanol (300 mL) and water (60 mL). The reaction solution was refluxed for 12 hours under nitrogen. After cooling to room temperature, the mixture was filtered through Celite. The filtrate concentrated to give the crude product, which was purified by HPLC C18 reverse phase column (mobile phase: acetonitrile/water/0.7% NH₄HCO₃, eluent gradient 10%-100% (volume ratio)). After the removal of volatile solvent, the desired fraction was lyophilized to give the title compound (19 g, yield: 23%).

### Step C:

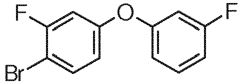

### 1 -bromo-2-fluoro-4-(3 -fluorophenoxy)benzene

### Procedure:

3-(3-fluoro-4-bromo-phenoxy)benzenamine (9 g, 32 mmol, 1.0 eq.) was added to pyridine-hydrogen fluoride solution (30 mL) in portions at -10 °C. The resulting reaction mixture was stirred at 0 °C for 30 minutes, cooled to -10 °C, and then sodium nitrite (2.42 g, 35 mmol, 1.1 eq.) was added portionwise. The reaction was stirred at 20 °C for 30 minutes, then at 60 °C for 14 hours. After cooling to room temperature, the mixture was poured into ice-ethanol (50 mL), diluted with a saturated solution of NaHCO₃ (50 mL), and then extracted with ethyl acetate (50 mL x 3). The combined organic phases were dried over anhydrous sodium sulfate and concentrated to give the crude product, which was purified by silica gel column chromatography (eluent: petroleum ether) to give the title compound (5.8 g, yield: 64%).

### Step D:

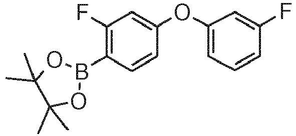

### 2-[2-fluoro-4-(3-fluorophenoxy)phenyl]-4,4,5,5-tetramethyl-1,3,2-dioxaborolane

### Procedure:

1-bromo-2-fluoro-4-(3-fluorophenoxy) benzene (5.8 g, 20 mmol, 1.0 eq.), bis pinacolato boronate (6.1 g, 24 mmol, 1.2 eq.), potassium acetate (3.9 g, 40 mmol, 2.0 eq.) and [1,1'-bis (diphenylphosphino) ferrocene] dichloropalladium (0.89 g, 1.2 mmol, 0.06 eq.) were dissolved in 1,4-dioxane (100 mL). The reaction mixture was stirred at 85 °C for 14 hours under a nitrogen atmosphere. After cooling to room temperature, the mixture was filtered through Celite. The filtrate was concentrated to give the crude product, which was purified by silica gel column chromatography (eluent: petroleum ether) to give the title compound (6.5 g, yield: 100%).

### Step E:

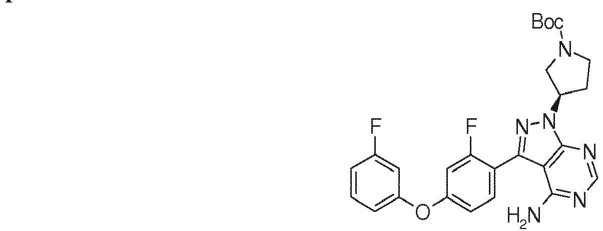

### (3R)-tert-butyl 3-[4-amino-3-[2-fluoro-4-(3-fluorophenoxy)phenyl]-1H-pyrazolo[3,4-d]pyrimidin-1-yl]pyrrolidine-1-carboxylate

### Procedure:

(R)-tert-butyl 3-(4-amino-3-iodo-1H-pyrazolo[3,4-d]pyrimidin-1-yl)pyrrolidine-1-carboxylate (6.5 g, 15.0 mmol, 1.0 eq.), 2-[2-fluoro-4-(3-fluorophenoxy)phenyl]-4,4,5,5-tetramethyl-1,3,2-dioxaborolane (6.5 g, 19.6 mmol, 1.3 eq.), potassium phosphate (6.4 g, 30.1 mmol, 2.0 eq.) and Pd-118 (0.25 g, 0.39 mmol, 0.01 eq.) were added to a mixture of 1,4-dioxane/water (16 mL, 1/1, v/v). The resulting mixture was stirred at 85 °C for 12 hours under a nitrogen atmosphere. After cooling to room temperature, the reaction mixture was diluted with water (50 mL), and then extracted with ethyl acetate (100 mL x 3). The combined organic phases were dried over anhydrous sodium sulfate and concentrated to give the crude product, which was purified by silica gel column chromatography (eluent: ethyl acetate) to give the title compound (4.2 g, yield: 55%).

### Step F:

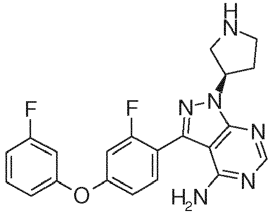

### 3-[2-fluoro-4-(3-fluorophenoxy)phenyl]-1-[(R)-pyrrolidin-3-yl]-1H-pyrazolo[3,4-d]pyrimidin-4-amine

### Procedure:

HCl/EA (10 mL, 4 mol/L) was added to a solution of (3R)-tert-butyl 3-[4-amino-3-[2-fluoro-4-(3-fluorophenoxy)phenyl]-1H-pyrazolo[3,4-d]pyrimidin-1-yl)pyrrolidine-1-carboxylate (4.2 g, 8.27 mmol) in dichloromethane (15 mL) at 0 °C. The reaction was stirred for 1 hour at room temperature, and then concentrated under reduced pressure to give the title compound hydrochloride (3.7 g, yield: 92%).

### Step G:

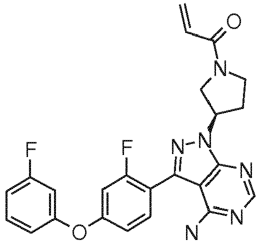

### 1-[(R)-3-[4-amino-3-[2-fluoro-4-(3-fluorophenoxy)phenyl]-1H-pyrazolo[3,4-d]pyrimidin-1-yl)pyrrolidin-1-yl)prop-1-en-1-one

### Procedure:

Sodium hydroxide (10%, 15.3 mL) and acryloyl chloride (0.67 g, 7.44 mmol, 0.9 eq.) were sequentially added dropwise to a solution of 3-[2-fluoro-4-(3-fluorophenoxy)phenyl]-1-[(R)-pyrrolidin-3-yl]-1H-pyrazolo[3,4-d]pyrimidin-4-amine (3.7 g, 8.27 mmol, 1.0 eq.) in tetrahydrofuran (20 mL) at 0 °C. The reaction was stirred at room temperature for 10 minutes, quenched with saturated NaHCO₃ (20 mL), and extracted with dichloromethane (30 mL x 3). The combined organic phases were dried over anhydrous sodium sulfate and concentrated to give the crude product, which was purified by silica gel column chromatography (eluent: petroleum ether: ethyl acetate = 1:0 to 1:1) to give the title compound (2.5 g, yield: 65%).
Spectroscopic data:
LC/MS (method: UFLC): RT = 3.178 min; m/z = 463.0 [M+H]⁺; Total running time = 7 min.
¹H NMR (400MHz, CDCl₃) δ 8.36 (s, 1H), 7.53-7.49 (m, 1H), 7.40-7.35 (m, 1H), 6.95-6.81 (m, 4H), 6.41-6.39 (m, 2H), 5.69-5.55 (m, 3H), 4.14-3.98 (m, 3H), 3.78-3.72 (m, 1H), 2.71-2.54 (m, 2H).

### Example 4

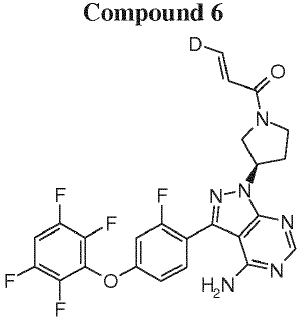

### (E)-1-[(R)-3-[4-amino-3-[2-fluoro-4-(3-fluorophenoxy)phenyl]-1H-pyrazolo[3,4-d]pyrimidin-1-yl]pyrrolidin-1-yl]-3-deuterium-prop-2-en-1-one

### Step A:

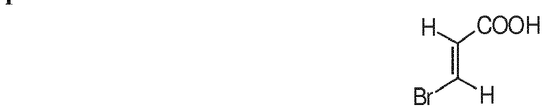

### (E)-3-bromoacrylic acid

### Procedure:

A mixture of propiolic acid (1 g, 14.28 mmol, 1.0 eq.) and HBr (40% aqueous solution, 1.7 mL, 0.88 eq.) was stirred overnight at 140 °C. Solvent was distilled off under reduced pressure. The obtained crude product was crystallized from water (4 mL x 3) to give the title compound (0.76 g, yield: 35%).
Spectroscopic data:
¹H NMR (400MHz, CDCl₃) δ 7.76 (d, *J* = 14 Hz, 1H), 6.55 (d, *J* = 14 Hz, 1H).

### Step B:

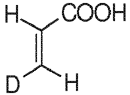

### (E)-3- deuteriumacrylic acid

### Procedure:

Na-Hg (6 g, 49.67 mmol, 2.5 eq.) was added to a solution of (*E*)-3-bromoacrylic acid (3 g, 19.87 mmol, 1.0 eq.) in D₂O (30 mL) at 0 ∼ 5 °C. The reaction was stirred at room temperature for 36 hours. The aqueous phase was adjusted pH = 5 with 1M hydrochloric acid, and then extracted with diethyl ether (20 mL x 5). The combined organic phases were dried over anhydrous sodium sulfate and concentrated under reduced pressure to give the title compound (0.52 g, yield: 36%).
Spectroscopic data:
¹H NMR (400MHz, CDCl₃) δ 7.76 (d, *J* = 17.2 Hz, 1H), 6.55 (d, *J*= 17.2 Hz, 1H).

### Step C:

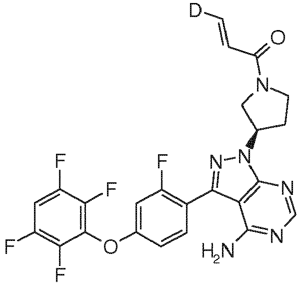

### (E)-1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]-3-deuterium-prop-2-en-1-one

### Procedure:

(*E*)-3-deuteriumacrylic acid (76 mg, 1.08 mmol, 1.0 eq.), HATU (530 mg, 1.40 mmol, 1.3 eq.) and N,N-diisopropylethylamine (419 mg, 3.24 mmol, 3.0 eq.) were added to a solution of 3-[2-fluoro-4-(2,3,5,6-tetrafluorophenoxy)phenyl]-1-[(R)-pyrrolidin-3-yl]-1H-pyrazolo[3,4-d]pyrimidin-4-amine (500 mg, 1.08 mmol, 1.0 eq.) in dichloromethane (50 mL). The reaction was stirred at room temperature for 12 hours, and concentrated to give the crude product, which was purified by HPLC-separation in a C18 reverse phase column (instrument: LC 8A & Gilson 215, fraction collector column: Synergi Max-RP 150*30mm*4u, mobile phase A: water (0.5% HCl), mobile phase B: acetonitrile, flow rate: 30 mL/min, gradient B: 36%∼37%, 0-17 minutes). After the removal of volatile solvent, the desired fraction was lyophilized to give the title compound hydrochloride (76 mg, yield: 13%).
Spectroscopic data:
LC/MS (method: UFLC): RT = 2.765 min; m/z = 518.1 [M+H]⁺; Total running time = 7 min.
¹H NMR (400MHz, CD₃OD) δ 8.41 (s, 1H), 7.66 (t, *J* = 8.4 Hz, 1H), 7.51-7.44 (m, 1H), 7.09-7.01 (m, 2H), 6.66-6.56 (m, 1H), 6.28-6.23 (m, 1H), 5.75-5.66 (m, 1H), 4.19-4.16 (m, 1H), 4.06-4.02 (m, 1.5H), 3.89-3.85 (m, 1H), 3.78-3.72 (m, 0.5H), 2.63-2.49 (m, 2H).

### Example 5

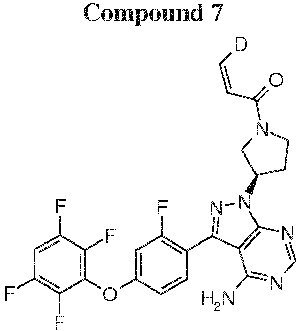

### (Z)-1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]-3-deuterium-prop-2-en-1-one

### Step A:

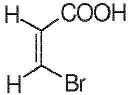

### (Z)-3-bromoacrylic acid

### Procedure:

A mixture of propiolic acid (1 g, 14.28 mmol, 1.0 eq.) and HBr (40% aqueous solution, 1.7 mL, 0.88 eq.) was stirred overnight at 55 °C. Solvent was distilled off under reduced pressure. The obtained crude product was crystallized from petroleum ether (4 mL x 3) to give the title compound (0.3 g, yield: 14%).
Spectroscopic data:
¹H NMR (400MHz, CDCl₃) δ 7.16 (d, *J=* 8.4 Hz, 1H), 6.67 (d, *J=* 8.4 Hz, 1H).

### Step B:

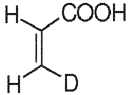

### (Z)-3- deuteriumacrylic acid

### Procedure:

Na-Hg (6 g, 49.67 mmol, 2.5 eq.) was added to a solution of (Z)-3-bromoacrylic acid (3 g, 19.87 mmol, 1.0 eq.) in D₂O (30 mL) at 0 ∼ 5 °C. The reaction was stirred at room temperature for 36 hours. The aqueous phase was adjusted pH = 5 with 1M hydrochloric acid, and then extracted with diethyl ether (20 mL x 5). The combined organic phases were dried over anhydrous sodium sulfate and concentrated under reduced pressure to give the title compound (0.34 g, yield: 23%).
Spectroscopic data:
¹H NMR (400MHz, CDCl₃) δ 6.14 (d, J= 10.4 Hz, 1H), 5.96 (d, *J* = 10.4 Hz, 1H).

### Step C:

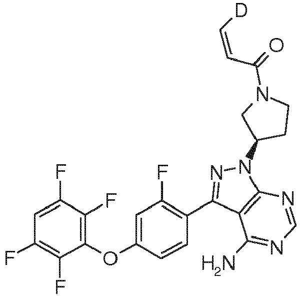

### (Z)-1-[(R)-3-[4-amino-3-[2-fluoro-4-(2,3,5,6-tetrafluorophenoxy)phenyl]-1H-pyrazolo[3,4-d]pyrimidin-1-yl]pyrrolidin-1-yl]-3-deuterium-prop-2-en-1-one

### Procedure:

(Z)-3-deuteriumacrylic acid (151 mg, 2.16 mmol, 1.0 eq.), HATU (1.06 g, 2.80 mmol, 1.3 eq.) and N,N-diisopropylethylamine (838 mg, 6.48 mmol, 3.0 eq.) were added to a solution of 3-[2-fluoro-4-(2,3,5,6-tetrafluorophenoxy)phenyl]-1-[(R)-pyrrolidin-3-yl]-1H-pyrazolo[3,4-d]pyrimidin-4-amine (1.0 g, 2.16 mmol, 1.0 eq.) in dichloromethane (50 mL). The reaction was stirred at room temperature for 12 hours, and concentrated to give the crude product, which was purified by HPLC-separation on a C18 reversed-phase column (instrument: LC 8A & Gilson 215, fraction collector column: Synergi Max-RP 150*30mm*4u, mobile phase A: water (0.5% HCl), mobile phase B: acetonitrile, flow rate: 30 mL/min, gradient B: 36%∼37%, 0∼17 minutes). After the removal of volatile solvent, the desired fraction was lyophilized to give the title compound hydrochloride (228 mg, yield: 20%).
Spectroscopic data:
LC/MS (method: UFLC): RT = 2.775 min; m/z = 518.1 [M+H]⁺; Total running time = 7 min.
¹H NMR (400MHz, CD₃OD) δ 8.45 (s, 1H), 7.70 (t, *J* = 8.4 Hz, 1H), 7.52-7.46 (m, 1H), 7.13-7.05 (m, 2H), 6.71-6.61 (m, 1H), 5.80-5.73 (m, 2H), 4.23-4.20 (m, 1H), 4.09-4.04 (m, 1.5H), 3.93-3.90 (m, 1H), 3.80-3.75 (m, 0.5H), 2.67-2.56 (m, 2H).

### Example 6

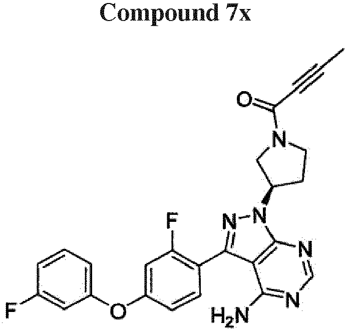

### 1-[(R)-3-[4-amino-3-[2-fluoro-4-(3-fluorophenoxy)phenyl]-1H-pyrazolo[3,4-d]pyrimidin-1-yl]pyrrolidin-1-yl]-butyl-2-yn-1-one

### Procedure:

2-butynoic acid (41.17 mg, 489.72 umol, 1.00 eq.), HATU (93.10 g, 244.86 umol, 0.50 eq.) and DIPEA (75.95 mg, 587.66 umol, 102.64 uL, 1.20 eq.) were added in sequence to a solution of 3-(2-fluoro-4-(3-fluorophenoxy)phenyl)-1-((R)-pyrrolidin-3-yl)-1H-pyrazolo[3,4-d]pyrimidin-4-amine (200.00 g, 489.72 umol, 1.00 eq.) in dichloromethane (5.0 mL). The reaction was stirred at the temperature of 15-18 °C for 2 hours, and then concentrated and dried in a rotary drier to obtain a crude product, which was purified by HPLC on a C18 reversed-phase column (mobile phase: acetonitrile/water/0.5% HCl, gradient elution 22% to 52% (volume ratio)). After removal of volatile components by evaporation under reduced pressure, the desired fraction was lyophilized to give hydrochloride of the title compound (82 mg, yield: 33%).
Spectroscopic data:
LC/MS (method: UFLC): RT = 3.057 min; m/z = 475.0 [M+H]⁺; Total running time = 7 min.
¹H NMR (400MHz, CDCl₃) δ 9.92 (s, 1H), 8.34 (d, *J* = 8.8 Hz, 1H), 7.56 (br, 1H), 7.41-7.36 (m, 1H), 7.00-6.86 (m, 5H), 6.58 (br, 1H), 5.62-5.58 (m, 1H), 4.22-3.74 (m, 4H), 2.65-2.50 (m, 2H), 2.02-1.96 (m, 3H).

### In Vitro Assay

### Inhibition Assay of BTK Kinase Activity:

The enzyme reaction mixture of BTK wild type standard HTRF assay contained 1 nM BTK wild type, 1 uM biotin-TKl peptide, 30 µM ATP, and 50 mM HEPES in a buffer. The enzyme reactions were carried out at room temperature for 60 minutes. 5 µl of 0.2 M EDTA were added to quench the reaction and then the inhibitors (5 L) were added at final concentrations of 2 nM antibody and 62.5 nM XL665. The plates were incubated at room temperature for 60 minutes and then read in the Envision plate reader. The readouts were transformed into inhibition rate% by the equation of (Min Ratio)/(Max-Min)*100%. Hence the IC50 data of test compounds were generated by using four parameter curve fitting.

### Inhibition Assay of Tumor Cell Activity:

Tumor cells (TMD-8, DoHH2, and WSU-DLCL2) were transferred and attached to 96-well plates. After one night, blank buffer and selected concentrations (0.01 nM-100 µM) of the test compound solution were added. After 48 hours incubation, CellTiter-Go was added to lyse the cells. Recordings of the luminescent signal and calculations of the percent inhibition of cell viability were taken.

### In Vivo Assay

A pharmacokinetic study in male SD rats: Male SD rats for pharmacokinetic study within 24 hours were divided into two groups: intravenous administration and oral administration. Each group has three animals. For the intravenous administration group, blood samples were collected at pre-dose, 0.0833, 0.167, 0.5, 1, 2, 4, 8, 24 h post-dose; for the oral administration group, blood samples were collected at pre-dose, 0.167, 0.5, 1, 2, 4, 8, 24 h post-dose. After blood collection, HPLC-MS/MS was applied to determine plasma concentrations of the compound. The calculated pharmacokinetic parameters of intravenous group include mean plasma clearance (CLp), mean apparent volume of distribution at steady state (Vdss), 0-24 h area under the curve (AUC), 0-24 h mean residence time (MRT), the half-life (T1/2); The calculated pharmacokinetic parameters of oral group include mean peak concentration (Cmax), 0-24 h area under the curve (AUC), 0-24 h mean residence time (MRT); mean relative bioavailability for the study.

A pharmacokinetic study in Beagle dogs: Beagle dogs for pharmacokinetic study within 24 hours were divided into two groups: intravenous administration (1 mg per kilogram) and oral administration (3 mg per kilogram). Each group has three animals. For the intravenous administration group, blood samples were collected at pre-dose, 0.033, 0.083, 0.25, 0.5, 1, 3, 6, 9, 24 h post-dose; for the oral administration group, blood samples were collected at pre-dose, 0.083, 0.25, 0.5, 1, 3, 6, 9, 24 h post-dose. After blood collection, HPLC-MS/MS was applied to determine plasma concentrations of the compound. The calculated pharmacokinetic parameters of intravenous group include mean plasma clearance (CLp), mean apparent volume of distribution at steady state (Vdss), 0-24 h area under the curve (AUC), 0-24 h mean residence time (MRT), the half-life (T1/2); The calculated pharmacokinetic parameters of oral group include mean peak concentration (Cmax), 0-24 h area under the curve (AUC), 0-24 h mean residence time (MRT); mean relative bioavailability for the study.

### Monotherapy and combination therapy in animals TMD-8, DoHH2, or WSU-DLCL2 inhibition of tumor model:

Experimental results show that the efficacy of co-administration of a synergistic effect on the sensitive (TMD-8), refractory (DoHH-2) as well as multi-drug resistance (WSU-DLCL2) tumor models have shown better than a single inhibitory effect for the drug.

A female CB-17SCID mouse was used in a xenograft model assessment compound (**3, 9, 14,** and other compounds listed in the table) and the joint anti-tumor effect of the medication was assessed. The TMD-8, DoHH2, WSU- DLCL2 tumor cells were seeded in 10% RPMI-1640 containing heat-inactivated fetal calf serum medium, and cultured at 37°C, 5% CO₂ conditions. Tumor cells were routinely subcultured twice a week. When cells grew into the exponential growth phase they were harvested and counted for tumor inoculation. The right of each mouse tumor cell was inoculated subcutaneously with 0.2 mL PBS suspension (10 x 10 6) and Matrigel (1/1). The mean tumor volume was approximately 100-200mm³ at the start of the administration. Each group consisted of a 6-10 mice-only composition. The test group (including the control group, single drug group, co-administration group) received the predetermined dose administered orally, which was continuously administered for 14 days or 21 days. During the experiment, tumor volume and body weight of mouse were measured once every two or three days.

### Collagen-induced arthritis model:

Male DBA/1 mice were used in *in vivo* collagen-induced arthritis model evaluation with Compound **3** and Compound **14** combinations to assess inhibitory effect. Mice were divided into 8 groups, including a control group, a solvent control group, and five treatment groups. All mice (except the normal group) at 0 and 21 days with 200µg bovine collagen (II type) immunity. Seven days after the booster immunization (28 days), the animals began to show symptoms of the disease, with an average clinical score of about 1. On the same day, immunized mice were randomly divided into seven groups: Compound **3** (1.5 mpk) and compound **14** (0.15 mpk) co-administered twice a day; Compound **3** (4.5mpk) and compound **14** (0.45mpk) administered in combination day twice; **14** (0.15 mpk) co-administered compound **3** (1.5 mpk) and the compound, once a day; compound **3** (1.5 mpk) monotherapy, once a day; compound **14** (1.5 mpk) monotherapy, once a day; positive control group (0.2 mg/kg dexamethasone), and the initiation of administration. Oral administration for two weeks, recording weight and clinical score. At the end of the study, animals were euthanized and hind limbs were collected for histopathological analysis.

### Adjuvant arthritis model:

Female Lewis rats were used for collagen-induced arthritis model in rats to assess compound **3** and compound **14** *in vivo* inhibitory effect of combination therapy. All rats (except control group) at day 0 left hind limb injection of complete Freund's adjuvant (CFA) for immunization. 6 days after immunization, some of the rats began to show clinical symptoms of arthritis, such as redness and swelling. At 13 days, the animals were re-immunized with a combination of seven groups: solvent control group, compound **3** (5 mpk) and compound **14** (0.5 mpk) co-administered twice a day; Compound **3** (15 mpk) and compound **14** (1.5 mpk) co-administered twice a day; **14** (3 mpk) co-administered compound **3** (30 mpk) and the compound, once a day; compound **3** (5 mpk) monotherapy, twice a day; compound **14** (0.5 mpk) monotherapy day two times; positive control group (compound 11, 3 mpk, twice a day), and start administration. Oral administration for three weeks, every other day record weight, clinical score and paw volume. At the end of the study, animals were sacrificed to collect the right hind paw, HE staining for histopathological analysis.

**Table 2. Data of BTK Activity Inhibition by the compounds in the examples of the present invention**

| Example | BTK IC₅₀ (µM) | Example | BTK IC₅₀ (µM) | Example | BTK IC₅₀ (µM) |
|---|---|---|---|---|---|
| **1** | 0.002 | **2** | 0.023 | **3** | 0.0005 |
| **4** | 0.021 | **5** | 0.001 | | |

**Table 3. Inhibition of individual compound on TMD-8 cell line (Inh%)**

| Compound | Compound Name (Mechanism) | Inh% | 100 uM | 10 uM | 1uM | 0.1uM | 0.01uM |
|---|---|---|---|---|---|---|---|
| **3** | **Compound 3** (BTK) | AVG | 99.69 | 74.93 | 61.66 | 59.59 | 46.07 |
| | | SD | 0.10 | 0.64 | 3.97 | 1.49 | 1.60 |
| **3** | **Compound 3** (BTK) | AVG | | | 55.05 | 52.40 | 51.66 |
| | | SD | | | 3.47 | 2.17 | 1.21 |
| **6** | **Compound 6** (BTK) | AVG | 99.38 | 62.52 | 60.21 | 52.99 | 32.29 |
| | | SD | 0.09 | 1.58 | 3.62 | 3.53 | 5.50 |
| **7** | **Compound 7** (BTK) | AVG | 99.32 | 62.89 | 58.57 | 58.68 | 33.85 |
| | | SD | 0.13 | 2.18 | 0.90 | 2.20 | 3.05 |
| **8** | Idelalisib (PI3K) | AVG | 96.79 | 87.69 | 68.92 | 48.83 | 29.44 |
| | | SD | 0.27 | 1.07 | 2.87 | 1.35 | 3.83 |
| **9** | Ibrutinib (BTK) | AVG | 99.93 | 81.41 | 66.11 | 59.14 | 56.16 |
| | | SD | 0.01 | 2.27 | 2.07 | 1.77 | 2.47 |
| **10** | Ruxolitinib (JAK1/2) | AVG | | | 100.78 | -5.53 | 0.05 |
| | | SD | | | 0.05 | 10.51 | 10.00 |
| **11** | Tofactinib (JAK3 ) | AVG | | | 6.66 | 1.31 | 6.02 |
| | | SD | | | 7.59 | 8.82 | 14.08 |
| **12** | ABT-199 Venetoclax (Bcl-2) | AVG | | | 37.55 | 18.81 | 11.12 |
| | | SD | | | 3.83 | 5.60 | 2.80 |
| **13** | OTS-964 (TOPK) | AVG | | | 101.20 | 49.10 | 8.71 |
| | | SD | | | 0.08 | 4.49 | 10.40 |
| **14** | Everolimus (mTOR) | AVG | | | 68.59 | 67.64 | 65.55 |
| | | SD | | | 1.71 | 2.76 | 2.35 |
| **15** | Pomalidomide (IMID) | AVG | | | 76.84 | 61.69 | 45.12 |
| | | SD | | | 1.03 | 2.34 | 1.26 |
| **16** | Lenalidomide (IMID) | AVG | | | 94.21 | 21.04 | 7.43 |
| | | SD | | | 0.46 | 5.67 | 2.61 |
| **17** | Rapamycin (mTOR) | AVG | | | 64.99 | 59.83 | 58.71 |
| | | SD | | | 2.77 | 1.45 | 2.37 |
| **18** | Methotrexate (antifolate) | AVG | | | 39.55 | 32.04 | -8.45 |
| | | SD | | | 0.71 | 3.05 | 6.02 |
| **19** | Ceritinib (ALK) | AVG | | | | | |
| | | SD | | | | | |

| | | | | | | | |
|---|---|---|---|---|---|---|---|
| **Note:** The compounds 3, 6, 7, **8-19** in this table correspond to the respective "Chinese or English compound names" as indicated, wherein compounds 3, 6 and 7 refer to the compounds in the examples of the present invention, while compounds 8-19 refer to the corresponding compounds in the prior art. For the sake of convenience for writing, an identifier number was given to a corresponding compound. Therefore, the identifier numbers of the compounds hereinafter will also have the same meaning. | | | | | | | |

**Table 4. Inhibition of "two in one" composition on TMD-8 cell line (Inh%)**

| Comp.@Conc. | Inh% | **3** @1 uM | **3** @0.1 uM | **3** @0.01 uM |
|---|---|---|---|---|
| **14** @0.1 uM | AVG | 65.94 | 67.20 | 66.17 |
| | SD | 1.41 | 0.73 | 1.64 |
| **15** @0.1 uM | AVG | 53.25 | 49.26 | 30.27 |
| | SD | 3.19 | 0.67 | 2.67 |
| **8** @0.1 uM | AVG | 68.05 | 64.71 | 63.56 |
| | SD | 2.04 | 2.50 | 5.10 |
| **13** @0.1 uM | AVG | 82.60 | 68.80 | 77.27 |
| | SD | 3.50 | 2.64 | 1.91 |
| **17** @0.1 uM | AVG | 75.73 | 80.41 | 75.12 |
| | SD | 0.53 | 1.29 | 6.22 |
| **12** @0.1 uM | AVG | 85.97 | 79.99 | 65.36 |
| | SD | 1.50 | 1.54 | 0.83 |
| **18** @0.1 uM | AVG | 59.93 | 46.58 | 35.68 |
| | SD | 2.77 | 6.76 | 5.94 |
| Comp.@Conc. | Inh% | **8** @1 uM | **8** @0.1 uM | **8** @0.01 uM |
| **14** @0.1 uM | AVG | 81.20 | 67.95 | 58.69 |
| | SD | 0.33 | 1.59 | 1.08 |
| **15** @0.1 uM | AVG | 76.96 | 42.58 | 24.14 |
| | SD | 0.95 | 7.50 | 3.94 |
| **13** @0.1 uM | AVG | 95.76 | 83.60 | 75.38 |
| | SD | 0.31 | 0.53 | 3.51 |
| **3** @0.1 uM | AVG | 86.26 | 80.21 | 73.01 |
| | SD | 2.25 | 2.87 | 2.46 |
| Comp. @Conc. | Inh% | **13** @1 uM | **13** @0.1 uM | **13** @0.01 uM |
| **8** @0.1 uM | AVG | 99.31 | 47.73 | 48.71 |
| | SD | 0.06 | 2.52 | 4.50 |
| **14** @0.1 uM | AVG | 99.46 | 59.47 | 60.30 |
| | SD | 0.11 | 0.73 | 1.44 |
| **15** @0.1 uM | AVG | 99.09 | 8.82 | 12.97 |
| | SD | 0.17 | 3.93 | 4.84 |
| **3** @0.1 uM | AVG | 99.16 | 97.60 | 52. 4 3 |
| | SD | 0.42 | 0.19 | 1.07 |

| Comp.@Conc. | Inh% | **15** @1 uM | **15** @0.1 uM | **15** @0.01 uM |
|---|---|---|---|---|
| **8** @0.1 uM | AVG | 80.19 | 43.34 | 42.81 |
| | SD | 1.25 | 5.76 | 3.81 |
| **14** @0.1 uM | AVG | 61.84 | 57.04 | 58.46 |
| | SD | 1.62 | 1.70 | 0.32 |
| **13** @0.1 uM | AVG | 71.75 | 30.72 | 1.36 |
| | SD | 0.35 | 7.16 | 5.17 |
| **3** @0.1 uM | AVG | 96.92 | 70.04 | 49.93 |
| | SD | 0.19 | 4.46 | 5.42 |
| Comp.@Conc. | Inh% | **18** @1 uM | **18** @0.1 uM | **18** @0.01 uM |
| **14** @0.1 uM | AVG | 54.43 | 52.67 | 56.87 |
| | SD | 0.70 | 2.71 | 2.27 |
| **3** @0.1 uM | AVG | 46.90 | 42.73 | 34.99 |
| | SD | 2.34 | 2.91 | 1.26 |
| Comp. @Conc. | Inh% | **9** @1 uM | **9** @0.1 uM | **9** @0.01 uM |
| **14** @0.1 uM | AVG | 71.04 | 58.89 | 56.54 |
| | SD | 2.52 | 9.71 | 13.33 |
| **19** @0.1 uM | AVG | 52.60 | 43.68 | 33.70 |
| | SD | 3.67 | 4.16 | 1.51 |
| **18** @0.1 uM | AVG | 55.19 | 42.42 | 32.13 |
| | SD | 2.63 | 3.32 | 3.08 |

It can be seen from Table 4 that the "two-in-one" pharmaceutical combinations demonstrated significant inhibition to tumor cell viability. Among them, the pharmaceutical combinations of compound 8 plus compound 13, compound 14 plus compound 13, compound 15 plus compound 13, and compound 3 plus compound 13 demonstrated highly effective inhibition to TMD-8 cell viability.

**Table 5. Inhibition of "three in one" composition on TMD-8 cell line (Inh%)**

| Comp.@Conc. | Inh% | **3** @1 uM | **3** @0.1 uM | **3** @0.01 uM |
|---|---|---|---|---|
| **14** @0.1 uM + **15** @0.1 uM | AVG | 76.42 | 80.77 | 83.22 |
| | SD | 4.50 | 1.38 | 0.37 |
| **17** @0.1 uM + **15** @ 0.1 uM | AVG | 89.89 | 85.62 | 88.57 |
| | SD | 0.72 | 7.68 | 3.37 |
| **14** @0.1 uM + **12** @0.1 uM | AVG | 93.44 | 94.73 | 94.65 |
| | SD | 0.55 | 0.92 | 1.11 |
| **8** @0.1 uM + **12** @0.1 uM | AVG | 95.56 | 95.30 | 94.62 |
| | SD | 0.40 | 0.10 | 0.06 |
| **18** @0.1 uM + **14** @0.1 uM | AVG | 66.44 | 71.70 | 58.27 |
| | SD | 8.75 | 1.91 | 2.80 |
| Comp.@Conc. | Inh% | **15** @1 uM | **15** @0. 1 uM | **15** @0.01 uM |
| **14** @0.1 uM + **3** @0.1 uM | AVG | 92.74 | 82.66 | 75.17 |
| | SD | 0.38 | 1.90 | 2.48 |
| Comp.@Conc. | Inh% | **8** @1 uM | **8** @0.1 uM | **8** @0.01 uM |
| **14** @0.1 uM + **15** @0.1 uM | AVG | 87.17 | 79.06 | 59.45 |
| | SD | 1.70 | 0.73 | 2.16 |
| Comp.@Conc. | Inh% | **13** @1 uM | **13** @0.1 uM | **13** @0.01 uM |
| **15** @0.1 uM + **3** @0.1 uM | AVG | 98.97 | 22.27 | -19.34 |
| | SD | 0.28 | 34.18 | 11.80 |
| **15** @0.1 uM + **8** @0.1 uM | AVG | 99.30 | 29.58 | -3.32 |
| | SD | 0.15 | 27.38 | 11.27 |
| **15** @0.1 uM + **14** @0.1 uM | AVG | 99.33 | 19.51 | -1.30 |
| | SD | 0.11 | 48.40 | 6.76 |
| Comp.@Conc. | Inh% | **10** @1 uM | **10** @0.1 uM | **10** @0.01 uM |
| **15** @0.1 uM + **3** @0.1 uM | AVG | 24.40 | -6.35 | -0.77 |
| | SD | 5.84 | 5.66 | 18.61 |
| **15** @0.1 uM + **8** @0.1 uM | AVG | 16.26 | 2.31 | -6.21 |
| | SD | 2.14 | 1.28 | 4.86 |
| **15** @0.1 uM + **14** @0.1 uM | AVG | -0.86 | 0.98 | -2.40 |
| | SD | 6.50 | 5.87 | 1.06 |
| Comp.@Conc. | Inh% | **9** @1 uM | **9** @0.1 uM | **9** @0.01 uM |
| **18** @0.1 uM + **14** @0.1 uM | AVG | 72.55 | 66.59 | 64.76 |
| | SD | 0.22 | 11.12 | 8.34 |
| **15** @0.1 uM + **14** @0.1 uM | AVG | 82.81 | 86.91 | 78.60 |
| | SD | 1.05 | 1.41 | 13.08 |

It can be seen from Table 5 that the "three-in-one" pharmaceutical combinations demonstrated significant inhibitory effect to tumor cell viability. Among them, the pharmaceutical combination of compound 3 plus compound 14 plus compound 12 and the pharmaceutical combination of compound 3 plus compound 8 plus compound 12 still demonstrated up to 95% high inhibitory effect to tumor cell viability even when concentration of BTK inhibitor was as low as 10nM.

**Table 6. Inhibition of individual compound and "three in one" composition on resistant WSU-DLCL2 cell line (Inh%)**

| Comp.@Conc. | Inh% | 1 uM | 0.1 uM | 0.01 uM |
|---|---|---|---|---|
| **3** | AVG | 41.04 | -1.36 | -10.06 |
| | SD | 7.73 | 2.59 | 11.14 |
| **15** | AVG | 46.61 | -8.32 | -14.53 |
| | SD | 1.15 | 4.49 | 13.72 |
| **14** | AVG | 55.35 | 46.71 | 40.81 |
| | SD | 1.67 | 0.53 | 2.67 |
| Comp.@Conc. | Inh% | **3** | **3** | **3** |
| **15** @0. 1 uM + **14** @0. 1 uM | AVG | 83.76 | 63.69 | 56.67 |
| | SD | 1.33 | 4.19 | 4.36 |

It can be seen from Table 6 that the "three-in-one" pharmaceutical combination of compound 3 plus compound 15 plus compound 14 demonstrated excellent inhibitory effect to cell viability of multi-drug tolerant WSU-DLCL2 cells, and was significantly better than each single-targeted drug.

**Table 7. Inhibition of individual compound and "three in one" composition on DoHH-2 cell line (Inh%)**

| Comp.@Conc. | Inh% | 1uM | 0.1uM | 0.01uM |
|---|---|---|---|---|
| **3** | AVG | 49.93 | 34.83 | 15.58 |
| | SD | 2.72 | 0.70 | 5.54 |
| **15** | AVG | 51.32 | 6.75 | -7.95 |
| | SD | 3.86 | 8.77 | 1.57 |
| **14** | AVG | 60.33 | 59.17 | 51.80 |
| | SD | 3.52 | 1.68 | 3.35 |
| Comp.@Conc. | Inh% | **3** | **3** | **3** |
| **15** @0. 1 uM + **14** @0.1 uM | AVG | 78.75 | 81.87 | 71.87 |
| | SD | 0.45 | 1.02 | 6.47 |

It can be seen from Table 7 that the "three-in-one" pharmaceutical composition of compound 3 plus compound 15 plus compound 14 demonstrated excellent inhibitory effect to cell viability of refractory DoHH-2 cells, and was significantly better than each single-targeted drug.

**Table 8. Inhibition of compositions with different proportions on TMD-8 cell line (Inh%)**

| Comp. ratio Comp.@Conc. | Inh% | 1.0 uM | 0.1 uM | 0.01 uM |
|---|---|---|---|---|
| **3** + **14** (19:1 molar ratio) | AVG | 72.97 | 71.71 | 66.64 |
| | SD | 0.93 | 1.49 | 0.83 |
| **3** + **14** + **15** (19:1:37 molar ratio) | AVG | 97.08 | 89.29 | 67.75 |
| | SD | 0.52 | 1.30 | 1.12 |
| **3** + **14** + **15** (1:1:1 molar ratio) | AVG | 97.16 | 91.23 | 80.09 |
| | SD | 0.17 | 0.85 | 0.96 |
| **3** + **14** + **15** (50:1:1 molar ratio) | AVG | 78.21 | 72.73 | 63.34 |
| | SD | 2.26 | 1.21 | 0.97 |
| **3** + **14** + **15** (10:1:1 molar ratio) | AVG | 88.09 | 77.18 | 68.76 |
| | SD | 0.70 | 1.66 | 2.12 |
| **14** @0.1 uM + **15** @0.1 uM | | **3** @ 1 uM | **3** @0.1 uM | **3** @0.01 uM |
| | AVG | 85.33 | 88.83 | 87.78 |
| | SD | 0.78 | 0.71 | 2.36 |

It can be seen from Table 8 that the "three-in-one" pharmaceutical combinations in various proportions demonstrated significant inhibitory effect to cell viability of TMD-8 cells.

**Table 9. PK parameters of compound 3 in rats**

| **Group** | 1 | | 2 | |
|---|---|---|---|---|
| **Dose Route** | IV | | PO | |
| **Dose level** | 2 mg/kg | | 10 mg/kg | |
| | AVG | SD | AVG | SD |
| **C₀ or Cₘₐₓ (ng/mL)** | 1390 | 247 | 641 | 191 |
| **Tₘₐₓ(hr)** | -- | -- | 1.33 | 0.753 |
| **T_{1/2}(hr)** | 0.787 | 0.0895 | 1.71 | 0.489 |
| **Vdss(L/kg)** | 1.61 | 0.339 | -- | -- |
| **CL(mL/min/Kg)** | 20.2 | 5.60 | -- | -- |
| **AUC_{0- last}(hr*ng/mL)** | 1740 | 421 | 3230 | 1120 |
| **AUC_{0- inf}(hr*ng/mL)** | 1740 | 420 | 3260 | 1140 |
| **Bioavailability(%)^{a}** | -- | -- | 37.1 | -- |

**Table 10. PK parameters of compound 3 in dogs**

| **Group** | 1 | | 2 | |
|---|---|---|---|---|
| **Dose Route** | IV | | PO | |
| **Dose level** | 2 mg/kg | | 5 mg/kg | |
| | AVG | SD | AVG | SD |
| **C₀ or Cₘₐₓ (ng/mL)** | 663 | 79.5 | 189 | 53.3 |
| **Tₘₐₓ(hr)** | -- | -- | 1.17 | 0.408 |
| **T_{1/2}(hr)** | 2.27 | 0.873 | 2.92 | 1.22 |
| **Vdss(L/kg)** | 4.24 | 0.370 | -- | -- |
| **CL(mL/min/Kg)** | 34.6 | 5.58 | -- | -- |
| **AUC_{0- last}(hr*ng/mL)** | 977 | 181 | 650 | 247 |
| **AUC_{0- inf}(hr*ng/mL)** | 987 | 183 | 574 | 123 |
| **Bioavailability(%)^{a}** | -- | -- | 26.2 | -- |

**Table 11. TK parameters of Compound 3 in rats**

| **Dose level (mg/kg)** | **Day** | **Gender** | **Cmax (ng/ml)** | **Tmax (h)** | **AUC₀₋₂₄ₕ (h*ng/mL)** |
|---|---|---|---|---|---|
| 40 | 1 | Male | 2160 | 2.0 | 13700 |
| | | Female | 2660 | 1.0 | 17300 |
| | 28 | Male | 2090 | 2.0 | 15400 |
| | | Female | 2970 | 1.0 | 17300 |
| 100 | 1 | Male | 2740 | 2.0 | 21700 |
| | | Female | 3700 | 4.0 | 28900 |
| | 28 | Male | 3990 | 2.0 | 30300 |
| | | Female | 3830 | 1.0 | 29600 |
| 200 | 1 | Male | 4220 | 2.0 | 37600 |
| | | Female | 4680 | 4.0 | 65200 |
| | 28 | Male | 4540 | 2.0 | 45100 |
| | | Female | 5490 | 8.0 | 60200 |

**Table 12. TK parameters of compound 3 in dogs**

| **Dose level (mg/kg)** | **Day** | **Gender** | **Cmax (ng/ml)** | **Tmax(h)** | **AUC₀₋₂₄ₕ (h*ng/mL)** |
|---|---|---|---|---|---|
| 15 | 1 | Male | 746 ± 18.1 | 2.0 (1.0-2.0) | 3550 ± 562 |
| | | Female | 685 ± 212 | 1.0 (1.0-2.0) | 2930 ± 980 |
| | 28 | Male | 576 ± 145 | 2.0 (2.0-2.0) | 3260 ± 732 |
| | | Female | 687 ± 123 | 2.0 (1.0-2.0) | 3730 ± 549 |
| 45 | 1 | Male | 1240 ± 381 | 2.0 (1.0-2.0) | 6480 ± 1670 |
| | | Female | 1220 ± 431 | 2.0 (2.0-2.0) | 6220 ± 3000 |
| | 28 | Male | 1470 ± 538 | 2.0 (2.0-4.0) | 9170 ± 3810 |
| | | Female | 1060 ± 263 | 2.0 (2.0-4.0) | 8130 ± 1490 |
| 105 | 28 | Male | 2700 ± 769 | 2.0 (2.0-2.0) | 16400 ± 5410 |
| | | Female | 2420 ± 670 | 2.0 (2.0-4.0) | 17300 ± 2830 |
| 150 | 1 | Male | 2460 ± 858 | 4.0 (1.0-8.0) | 22900 ± 13900 |
| | | Female | 1850 ± 605 | 2.0 (1.0-4.0) | 11200 ± 5990 |

**Table 13: Inhibitory effect of administration of an individual drug and pharmaceutical combinations on tumor cells in an animal tumor model**

| **Figures** | **Course of treatment** | **Compound (mg/kg)** | **Tumor inhibition (%)** |
|---|---|---|---|
| Figure 1 | Gavage, twice a day, 14 days | Control group | -- |
| | | **1** (10 mg/kg) | 56 |
| | | **1** (30 mg/kg) | 77 |
| | | **3** (10 mg/kg) | 64 |
| | | **3** (30 mg/kg) | 82 |
| | | **3** (90 mg/kg) | 93 |
| Figure 2 | Gavage, twice a day, 14 days | Control group | -- |
| | | **3** (10 mg/kg) | 63 |
| | | **3** (30 mg/kg) | 89 |
| | | **15** (30 mg/kg) | 24 |
| | | **3** (10 mg/kg) | 95 |
| | | **15** (30 mg/kg) | |
| | | Control group | -- |
| | | **3** (5 mg/kg) | 90 |
| | | **3** (10 mg/kg) | 96 |
| | | **15** (10 mg/kg) | -8 |
| | | **8** (10 mg/kg) | 26 |
| | | **3** (5 mg/kg) | 82 |
| | | **15** (10 mg/kg) | |
| Figure 3 | Gavage, twice a day, | **3** (10 mg/kg) | 89 |
| | 21 days | **15** (10 mg/kg) | |
| | | **3** (5 mg/kg) | 95 |
| | | **8** (10 mg/kg) | |
| | | **3** (10 mg/kg) | 98 |
| | | **8** (10 mg/kg) | |
| | | **3** (5 mg/kg) | 94 |
| | | **15** (10 mg/kg) | |
| | | **8** (5 mg/kg) | |
| | | **3** (10 mg/kg) | 94 |
| | | **15** (10 mg/kg) | |
| | | **8** (10 mg/kg) | |
| | | **15** (10 mg/kg) | 86 |
| | | **8** (10 mg/kg) | |
| | | Control group | - |
| | | **3** (10 mg/kg) | 77 |
| | | **16** (10 mg/kg) | 16 |
| | | **16** (30 mg/kg) | 42 |
| | | **14** (1 mg/kg) | 97 (tumor growth rebound on day 17) |
| Figure 4 | Gavage, twice a day, | **14** (3 mg/kg) | 99 (tumor growth rebound on day 19) |
| | 21 days | **3** (10 mg/kg) | 100 (no tumor growth rebound after day 15) |
| | | **14** (1 mg/kg) | |
| | | **3** (10 mg/kg) | 100 (no tumor growth rebound after day 15) |
| | | **14** (3 mg/kg) | |
| | | **3** (10 mg/kg) | 72 |
| | | **16** (10 mg/kg) | |
| | | **3** (10 mg/kg) | 80 |
| | | **16** (30 mg/kg) | |
| Figure 5 | Gavage, twice a day, 21 days | Control group | - |
| | | **3** (5 mg/kg) | 33 |
| | | **3** (5 mg/kg) | 100 (tumor completely disappeared on day 9, no rebound) |
| | | **15** (5 mg/kg) | |
| | | **14** (0.5 mg/kg) | |
| Figure 10 | Gavage, twice a day, 14 days | Control group | - |
| | | **3** (5 mg/kg) | 100 (tumor completely disappeared on day 10, no rebound) |
| | | **15** (5 mg/kg) | |
| | | **14** (0.5 mg/kg) | |
| | | **3** (10 mg/kg) | 100 (tumor completely disappeared on day 10, no rebound) |
| | | **15** (1 mg/kg) | |
| | | **14** (0.5 mg/kg) | |
| | | **3** (20 mg/kg) | 100 (tumor completely disappeared on day 10, no rebound) |
| | | **15** (1 mg/kg) | |
| | | **14** (0.5 mg/kg | |
| Figure 7 | Gavage, twice a day, 21 days | Control group | -- |
| | | **3** (5 mg/kg) | 15.9 |
| | | **3** (5 mg/kg) | 80.3 |
| | | **15** (5 mg/kg) | |
| | | **14** (0.5 mg/kg | |
| Figure 6 | Gavage, twice a day, 21 days | Control group | -- |
| | | **3** (5 mg/kg) | 28.8 |
| | | **3** (10 mg/kg) | 20.1 |
| | | **3** (30 mg/kg) | 35.6 |
| | | **3** (5 mg/kg) | 58.3 |
| | | **14** (0.5 mg/kg | |
| | | **3** (5 mg/kg) | 79.4 |
| | | **15** (5 mg/kg) | |
| | | **14** (0.5 mg/kg | |
| Figure 8 | Gavage, twice a day, 28 days | Control group | -- |
| | | **9** (30 mg/kg) | 24 |
| | | **3** (10 mg/kg) | 22 |
| | | **3** (30 mg/kg) | 30 |
| | | **3** (45 mg/kg) | 32 |
| Figure 9 | Gavage, twice a day, 18 days | Control group | -- |
| | | **3** (5 mg/kg) | 4 |
| | | **3** (10 mg/kg) | 7 |
| | | **3** (30 mg/kg) | 23 |
| | | **3** (5 mg/kg) | 44 |
| | | **14** (0.5 mg/kg) | |
| | | **3** (5 mg/kg) | 50 |
| | | **15** (5 mg/kg) | |
| | | **14** (0.5 mg/kg) | |
| Figure 11 | Gavage, twice a day, 14 days | Control group | -- |
| | | **3** (5 mg/kg) | 63 |
| | | **15** (5 mg/kg) | |
| | | **14** (0.5 mg/kg) | |
| | | **9** (4.3 mg/kg) | 67 |
| | | **15** (5 mg/kg) | |
| | | **14** (0.5 mg/kg) | |
| | | Control group | -- |
| | | **3** (5 mg/kg) | 75 |
| | | **12** (5 mg/kg) | 12 |
| | | **8** (10 mg/kg) | 48 |
| | | **3** (5 mg/kg) | 86 |
| | | **12** (5 mg/kg) | |
| | | **8** (10 mg/kg) | 37 |
| Figure 12 | Gavage, twice a day, | **12** (5 mg/kg) | |
| | 14 days | **12** (5 mg/kg) | 77 |
| | | **14** (0.5 mg/kg) | |
| | | **3** (5 mg/kg) | 100 |
| | | **12** (5 mg/kg) | |
| | | **14** (0.5 mg/kg) | |
| | | **3** (5 mg/kg) | 89 |
| | | **12** (5 mg/kg) | |
| | | **8** (10 mg/kg) | |

It can be seen from Table 13 that drug administration in combination had synergistic effect and additive lethal capacity on tumor cells. Therapeutic effect of administration in combination was far better than each single-targeted drug. For example, administration of the "two-in-one" combination of compound 3 plus compound 14 could result in complete disappearance of tumor cells in a treatment period of 15 days. Meanwhile, administration of the "three-in-one" combination of compound 3 plus compound 14 plus compound 15 could result in complete disappearance of tumor cells in a shorter treatment period (9 days), and no tumor growth rebound was observed in 12 days after administration was stopped, indicating a significantly better therapeutic effect than single-targeted drugs.

**Table 14. Influence of administration of pharmaceutical combinations on body weight in an animal tumor model**

| **Animal weight (g)** | **Time (day)** | **0** | **2** | **5** | **7** | **9** | **12** | **14** |
|---|---|---|---|---|---|---|---|---|
| Control group | AVG | 22.9 | 22.4 | 22.8 | 23.0 | 23.7 | 23.5 | 23.6 |
| | SD | 0.5 | 0.5 | 0.6 | 0.5 | 0.6 | 0.5 | 0.6 |
| **3** (5 mg/kg) | AVG | 21.9 | 21.6 | 22.8 | 22.5 | 22.6 | 22.5 | 22.3 |
| **15** (5 mg/kg) | SD | 0.4 | 0.4 | 0.3 | 0.4 | 0.5 | 0.5 | 0.6 |
| **14** (0.5 mg/kg) | | | | | | | | |
| **9** (4.3 mg/kg) | AVG | 22.2 | 21.7 | 22.7 | 22.9 | 23.1 | 22.7 | 22.6 |
| **15** (5 mg/kg) | SD | 0.5 | 0.5 | 0.6 | 0.5 | 0.4 | 0.4 | 0.6 |
| **14** (0.5 mg/kg) | | | | | | | | |

It can be seen from Table 14 that animals in different groups showed no significant difference in body weight, indicating it was safe to administer drug combinations at low dose levels.

**Table 15. Influence of administration of a pharmaceutical combination 3/14/15 on body weight in an animal tumor model**

| **Animal weight (g)** | **Time (day)** | **0** | **2** | **5** | **7** | **9** | **12** | **14** |
|---|---|---|---|---|---|---|---|---|
| Control group | AVG | 21.2 | 21.1 | 21.1 | 21.4 | 21.5 | 21.8 | 21.9 |
| | SD | 0.5 | 0.4 | 0.4 | 0.5 | 0.4 | 0.5 | 0.5 |
| **3** (5 mg/kg) | AVG | 22.1 | 21.8 | 21.8 | 21.8 | 21.7 | 22.3 | 21.7 |
| **15** (5 mg/kg) | SD | 0.8 | 0.7 | 0.8 | 0.7 | 0.8 | 0.8 | 0.7 |
| **14** (0.5 mg/kg) | | | | | | | | |
| **3** (10 mg/kg) | AVG | 21.6 | 21.5 | 21.8 | 22.2 | 22.4 | 22.4 | 22.1 |
| **15** (1 mg/kg) | SD | 0.5 | 0.6 | 0.6 | 0.7 | 0.7 | 0.6 | 0.7 |
| **14** (0.5 mg/kg) | | | | | | | | |
| **3** (20 mg/kg) | AVG | 21.4 | 21.0 | 21.1 | 21.3 | 21.4 | 21.3 | 21.4 |
| **15** (1 mg/kg) | SD | 0.6 | 0.5 | 0.5 | 0.6 | 0.6 | 0.5 | 0.6 |
| **14** (0.5 mg/kg) | | | | | | | | |

It can be seen from Table 15 that animals in different groups showed no significant difference in body weight, indicating it was safe to administer drug combinations at low dose levels.

**Table 16. Foot volume - Arthritis induced by adjuvant**

| **Foot volume (mL)** | **Time (day)** | **0** | **17** | **26** | **28** | **31** | **33** |
|---|---|---|---|---|---|---|---|
| Regular group | AVG | 1.00 | 1.08 | 1.05 | 1.05 | 1.05 | 1.04 |
| | SD | 0.03 | 0.02 | 0.01 | 0.02 | 0.02 | 0.02 |
| Solvent control | AVG | 1.12 | 2.18 | 2.71 | 2.71 | 2.69 | 2.70 |
| | SD | 0.09 | 0.09 | 0.15 | 0.11 | 0.11 | 0.11 |
| **3** (5 mg/kg) | AVG | 1.03 | 1.77* | 1.65** * | 1.69** * | 1.63** * | 1.53** * |
| **14** (0.5mg/kg) | SD | 0.02 | 0.07 | 0.08 | 0.08 | 0.08 | 0.07 |
| **3** (15 mg/kg) | AVG | 1.01 | 1.62** * | 1.45** * | 1.44** * | 1.34** * | 1.29** * |
| **14** (1.5mg/kg) | SD | 0.02 | 0.10 | 0.11 | 0.10 | 0.09 | 0.08 |
| **3** (30 mg/kg) | AVG | 1.01 | 1.63** * | 1.50** * | 1.45** * | 1.41** * | 1.38** * |
| **14** (3mg/kg) | SD | 0.01 | 0.08 | 0.09 | 0.08 | 0.08 | 00.7 |
| **3** (5 mg/kg) | AVG | 1.04 | 1.87ns | 2.29** | 2.12** * | 2.12** * | 2.09** * |
| | SD | 0.01 | 0.12 | 0.14 | 0.19 | 0.19 | 0.20 |
| **14** (0.5 mg/kg) | AVG | 1.02 | 1.84ns | 2.01** * | 2.04** * | 1.94** * | 1.93** * |
| | SD | 0.01 | 0.08 | 0.13 | 0.12 | 0.11 | 0.10 |
| **11** (3 mg/kg) | AVG | 1.00 | 1.54** * | 1.37** * | 1.33** * | 1.23** * | 1.23** * |
| | SD | 0.02 | 0.08 | 0.08 | 0.07 | 0.05 | 0.05 |
| *p<0.05, **p<0.01, ***p<0.001 | | | | | | | |

It can be seen from Table 16 that the two-in-one pharmaceutical combination was more effective than individual drugs.

**Table 17. Pathological score - Arthritis induced by adjuvant**

| Groups | Pathological score (AVG ± SD) | | | | |
|---|---|---|---|---|---|
| | Inflammatory cell infiltration | Pannus formation | Cartilage injury | Bone resorption | Total score |
| Regular group | 0.0±0.00 | 0.0±0.00 | 0.0±0.00 | 0.0±0.00 | 0.0±0.00 |
| Solvent control | 4±0.00 | 4±0.00 | 3.8±0.13 | 3.7±0.15 | 15.5±0.27 |
| **3** (5 mg/kg) | 3.5±0.22 | 2.8±0.25 | 2.6±0.27 | 2.8±0.29 | 11.7±0.96 |
| **14** (0.5 mg/kg) | | | | | |
| **3** (5 mg/kg) | 2.9±0.31 | 1.6±0.22 | 1.1±0.28 | 2.2±0.49 | 7.8±1.21*** |
| **14** (1.5 mg/kg) | | | | | |
| **3** (30 mg/kg) | 2.6±0.37 | 2.2±0.44 | 1.7±0.40 | 2.4±0.40 | 8.9±1.55*** |
| **14** (3 mg/kg) | | | | | |
| **3** (5 mg/kg) | 3.5±0.34 | 3.2±0.53 | 2.8±0.51 | 3.0±0.45 | 12.5±1.78 |
| **14** (0.5 mg/kg) | 3.9±0.10 | 3.7±0.21 | 3.5±0.27 | 3.5±0.17 | 146±0.54 |
| **11** (3 mg/kg) | 1.9±0.18 | 0.4±0.22 | 0.2±0.20 | 0.5±0.22 | 3.0±0.73*** |
| ***p<0.001, vs. blank control, post-hoc test | | | | | |

It can be seen from Table 17 that the "two-in-one" pharmaceutical combination was more effective than individual drugs.

**Table 18. Average score of clinical evaluation from day 21 after initial immunization - Arthritis induced by collagen**

| **Clinical score** | **Time (day)** | **21** | **32** | **39** | **42** |
|---|---|---|---|---|---|
| Regular group | AVG | 0.00 | 0.00 | 0.00 | 0.00 |
| | AD | 0.00 | 0.00 | 0.00 | 0.00 |
| Blank control | AVG | 0.00 | 4.00 | 7.6 | 8.00 |
| | AD | 0.00 | 0.86 | 1.14 | 1.20 |
| Dexamethasone (0.2 mg/kg) | AVG | 0.00 | 0.60*** | 0.40*** | 0.20*** |
| | AD | 0.00 | 0.34 | 0.22 | 0.20 |
| **3** (1.5 mg/kg) | AVG | 0.00 | 1.60* | 1.40*** | 1.60*** |
| **14** (0.15 mg/kg) Twice a day | AD | 0.00 | 0.45 | 0.37 | 0.40 |
| **3** (4.5 mg/kg) | AVG | 0.00 | 0.40*** | 0.2*** | 0.10*** |
| **14** (0.45 mg/kg) Twice a day | AD | 0.00 | 0.16 | 0.13 | 0.10 |
| **3** (1.5 mg/kg) | AVG | 0.00 | 1.50*** | 1.40*** | 1.60*** |
| **14** (0.15 mg/kg) Once a day | AD | 0.00 | 0.40 | 0.50 | 0.58 |
| **3** (0.15 mg/kg) Once a day | AVG | 0.00 | 2.60 | 4.20*** | 4.00*** |
| | AD | 0.00 | 0.86 | 1.14 | 1.22 |
| **14** (0.15 mg/kg) Once a day | AVG | 0.00 | 1.00 | 5.60 | 5.70* |
| | AD | 0.00 | 0.54 | 0.82 | 0.80 |
| *p<0.05, **p<0.01, ***p<0.001, vs. blank control, Two-way ANOVA, Bonferrni's post-hoc test | | | | | |

It can be seen from Table 18 that the "two-in-one" pharmaceutical combinations are more effective than individual drugs.

**Table 19. Pathological score - Arthritis induced by collagen**

| Groups | Analyzed body part | Pathological score (Mean ± SEM) | | | | |
|---|---|---|---|---|---|---|
| | | Inflammatory cell infiltration | Pannus formation | Cartilage injury | Bone resorption | Total score |
| Blank control | Left hind legs | 1.60±0.65 | 1.30±0.56 | 1.40±0.58 | 1.00±0.42 | 15.50±2.30 |
| | Right hind legs | 2.80±0.61 | 2.40±0.54 | 2.50±0.56 | 2.50±0.56 | |
| Dexamethasone (0.2 mg/kg) Once a day | Left hind legs | 0.00±0.00 | 0.00±0.00 | 0.00±0.00 | 0.00±0.00 | 0.00±0.00* ** |
| | Right hind legs | 0.00±0.00 | 0.00±0.00 | 0.00±0.00 | 0.00±0.00 | |
| **3** (1.5 mg/kg) | Left hind legs | 0.50±0.22 | 0.20±0.20 | 0.20±0.20 | 0.10±0.10 | 1.50±0.78* ** |
| **14** (0.15 mg/kg) Twice a day | Right hind legs | 0.20±0.20 | 0.10±0.10 | 0.10±0.10 | 0.10±0.10 | |
| **3** (4.5 mg/kg) | Left hind legs | 0.00±0.00 | 0.00±0.00 | 0.00±0.00 | 0.00±0.00 | 0.00±0.00* ** |
| **14** (0.45 mg/kg) Twice a day | Right hind legs | 0.00±0.00 | 0.00±0.00 | 0.00±0.00 | 0.00±0.00 | |
| **3** (1.5 mg/kg) | Left hind legs | 0.20±0.20 | 0.20±0.20 | 0.20±0.20 | 0.20±0.20 | 0.80±0.80* ** |
| **14** (0.15 mg/kg) Once a day | Right hind legs | 0.00±0.00 | 0.00±0.00 | 0.00±0.00 | 0.00±0.00 | |
| **3** (0.15 mg/kg) Once a day | Left hind legs | 1.00±0.47 | 0.80±0.47 | 0.80±0.47 | 0.60±0.13 | 3.90±1.80* ** |
| | Right hind legs | 0.20±0.20 | 0.20±0.20 | 0.20±0.20 | 0.10±0.10 | |
| **14** (0.15 mg/kg) Once a day | Left hind legs | 2.10±0.64 | 2.00±0.67 | 2.00±0.67 | 1.80±0.61 | 15.90±4.50 |
| | Right hind legs | 2.00±0.67 | 2.00±0.67 | 2.00±0.67 | 2.00±0.67 | |
| Regular group | Left hind legs | 0.00±0.00 | 0.00±0.00 | 0.00±0.00 | 0.00±0.00 | 0.00±0.00* ** |
| | Right hind legs | 0.00±0.00 | 0.00±0.00 | 0.00±0.00 | 0.00±0.00 | |
| ***p<0.001, vs. blank control, Kruskal-Wallis test, Dunn's post-hoc test | | | | | | |

It can be seen from Table 19 that the "two-in-one" pharmaceutical combinations are more effective than individual drugs.

## Claims

1. A compound represented by Formula (I), (II), (Ia), (IIa), (Ib) or (IIb), or an enantiomer, diastereomer or pharmaceutically acceptable salt thereof; 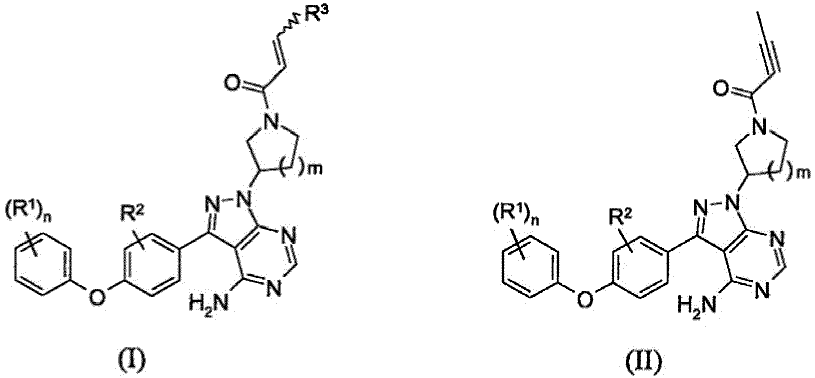 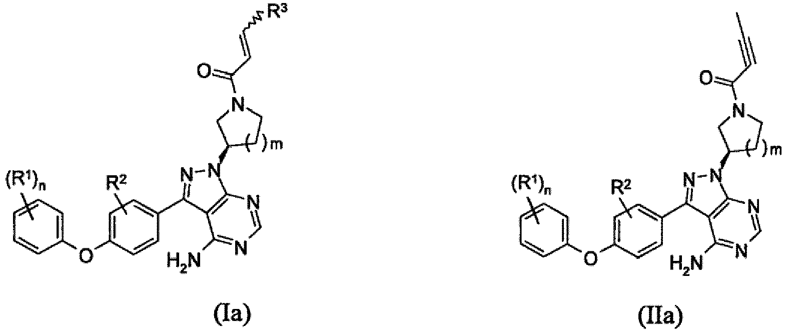 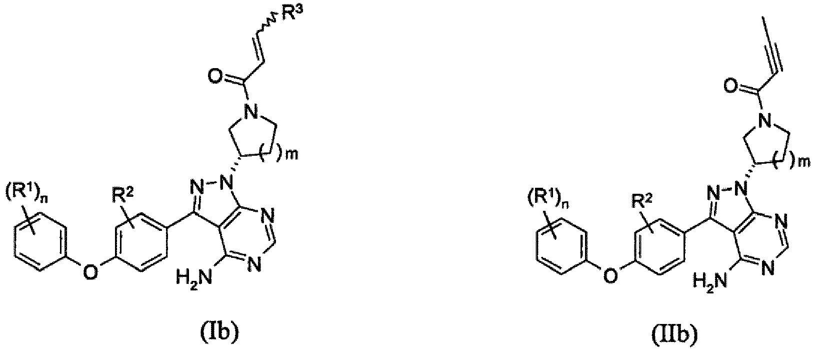 Wherein:
R¹ is F; n is 1, 2, 3 or 4;
R² is F; m is 1 or 2
R³ is H or D.

2. A compound selected from the table below, or a racemate or pharmaceutically acceptable salt thereof;

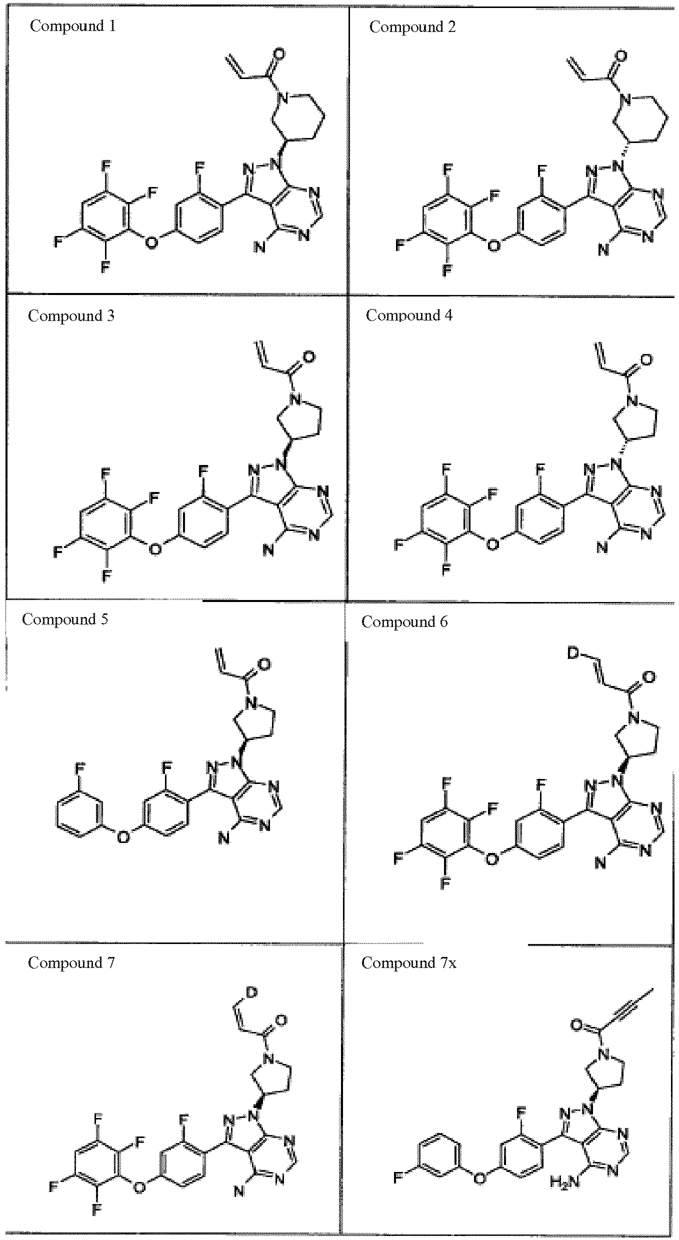

3. A pharmaceutical composition comprising an inactive carrier and a compound of claim 1 or 2 or a pharmaceutically acceptable salt thereof, preferably a compound of claim 2.

4. A medicament comprising an inactive carrier and a compound of claim 1 or 2, preferably a compound of claim 2, or a pharmaceutically acceptable salt thereof, as an active ingredient.

5. A method of inhibiting BTK activity in a patient, comprising administering to the patient a therapeutically effective amount of a compound of claim 1 or 2.

6. A method of treating or inhibiting an autoimmune disease or disorder, comprising administering to a patient in need thereof a therapeutically effective amount of a compound of claim 1 or 2 or a pharmaceutical composition of claim 3, wherein the autoimmune disease includes, but is not limited to, an organ-specific autoimmune disease such as chronic lymphocytic thyroiditis, hyperthyroidism, insulin-dependent diabetes mellitus, myasthenia gravis, chronic ulcerative colitis, pernicious anemia associated with chronic atrophic gastritis, Goodpasture's syndrome, pemphigus vulgaris, pemphigoid, primary biliary cirrhosis, multiple cerebrospinal sclerosis, acute idiopathic neuritis, etc.; a systemic autoimmune disease such as systemic lupus erythematosus, rheumatoid arthritis, psoriasis, systemic vasculitis, scleroderma, pemphigus, mixed connective tissue disease, autoimmune hemolytic anemia, autoimmune thyroid disease, ulcerative colitis, etc.

7. A method of treating or inhibiting an immune disorder, comprising administering to a patient in need thereof a therapeutically effective amount of a compound of claim 1 or 2 or a pharmaceutical component of claim 3, wherein the immune disorder includes, but is not limited to, serum sickness, asthma, allergic rhinitis, drug allergy, etc.

8. A method of treating or inhibiting an inflammatory disease or disorder, comprising administering to a patient in need thereof a therapeutically effective amount of a compound of claim 1 or 2 or a pharmaceutical composition of claim 3, wherein the inflammatory disease includes, but is not limited to, keratitis, rhinitis, stomatitis, mumps, pharyngitis, tonsillitis, tracheitis, bronchitis, pneumonia, myocarditis, gastritis, gastroenteritis, cholecystitis, appendicitis, etc.

9. A method of treating or inhibiting cancer or other diseases, comprising administering to a patient in need thereof a therapeutically effective amount of a compound of claim 1 or 2 or a pharmaceutical composition of claim 3, wherein the cancer or other diseases include, but are not limited to, various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL) and multiple myeloma (MM)) and other diseases that would benefit from inhibition of BTK activity.

10. Use of a compound of claim 1 or 2 for the manufacture of a medicament for treating or inhibiting an autoimmune disease or disorder, wherein the autoimmune disease includes, but is not limited to, an organ-specific autoimmune disease such as chronic lymphocytic thyroiditis, hyperthyroidism, insulin-dependent diabetes mellitus, myasthenia gravis, chronic ulcerative colitis, pernicious anemia associated with chronic atrophic gastritis, Goodpasture's syndrome, pemphigus vulgaris, pemphigoid, primary biliary cirrhosis, multiple cerebrospinal sclerosis, acute idiopathic neuritis, etc.; a systemic autoimmune disease such as systemic lupus erythematosus, rheumatoid arthritis, psoriasis, systemic vasculitis, scleroderma, pemphigus, mixed connective tissue disease, autoimmune hemolytic anemia, autoimmune thyroid disease, ulcerative colitis, etc.

11. Use of a compound of claim 1 or 2 for the manufacture of a medicament for treating or inhibiting an immune disorder, wherein the immune disorder includes, but is not limited to, serum sickness, asthma, allergic rhinitis, drug allergy, etc.

12. Use of a compound of claim 1 or 2 for the manufacture of a medicament for treating or inhibiting an inflammatory disease or disorder, wherein the inflammatory disease includes, but is not limited to, keratitis, rhinitis, stomatitis, mumps, pharyngitis, tonsillitis, tracheitis, bronchitis, pneumonia, myocarditis, gastritis, gastroenteritis, cholecystitis, appendicitis, etc.

13. Use of a compound of claim 1 or 2 for the manufacture of a medicament for treating or inhibiting cancer or other diseases, wherein the cancer or other diseases include, but are not limited to various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL) and multiple myeloma (MM)) and other diseases that would benefit from inhibition of BTK activity.

14. Use of a composition comprising a compound of claim 1 or 2 and various CD20 antibodies for the manufacture of a medicament for treating or inhibiting cancer or other diseases, wherein the cancer or other diseases include, but are not limited to various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL) and multiple myeloma (MM)) and other diseases that would benefit from inhibition of BTK activity.

15. A "three-in-one" pharmaceutical composition comprising a BTK inhibitor, an mTOR inhibitor and an immunomodulatory drug (IMiD).

16. The "three-in-one" pharmaceutical composition of claim 15 comprising BTK inhibitor ibrutinib, mTOR inhibitor everolimus and an IMiD such as thalidomide/revlimid/pomalidomide/CC-122/CC-220.

17. The "three-in-one" pharmaceutical composition of claim 15 comprising Compound 3 of claim 2, mTOR inhibitor everolimus, and an IMiD such as thalidomide/revlimid/pomalidomide/CC-122/CC-220.

18. The "three-in-one" pharmaceutical composition of claim 15 comprising Compound **5** of claim 2, mTOR inhibitor everolimus and an IMiD such as thalidomide/revlimid/pomalidomide/CC-122/CC- 220.

19. The "three-in-one" pharmaceutical composition of claim 15 comprising BTK inhibitor ibrutinib, mTOR inhibitor rapamycin and an IMiD such as thalidomide/revlimid/pomalidomide/CC-122/CC-220.

20. The "three-in-one" pharmaceutical composition of claim 15 comprising Compound **3** of claim 2, mTOR inhibitor rapamycin and an IMiD such as thalidomide/revlimid/pomalidomide/CC-122/CC-220.

21. The "three-in-one" pharmaceutical composition of claim 15 comprising Compound **5** of claim 2, mTOR inhibitor rapamycin and an IMiD such as thalidomide/revlimid/pomalidomide/CC-122/CC-220.

22. A "three-in-one" pharmaceutical composition comprising a BTK inhibitor, an mTOR inhibitor and a Bcl-2 inhibitor.

23. The "three-in-one" pharmaceutical composition of claim 22 comprising BTK inhibitor ibrutinib, mTOR inhibitor everolimus and Bcl-2 inhibitor ABT-199.

24. The "three-in-one" pharmaceutical composition of claim 22 comprising Compound **3** of claim 2, mTOR inhibitor everolimus and Bcl-2 inhibitor ABT-199.

25. The "three-in-one" pharmaceutical composition of claim 22 comprising Compound **5** of claim 2, mTOR inhibitor everolimus and Bcl-2 inhibitor ABT-199.

26. The "three-in-one" pharmaceutical composition of claim 22 comprising BTK inhibitor ibrutinib, mTOR inhibitor rapamycin and Bcl-2 inhibitor ABT-199.

27. The "three-in-one" pharmaceutical composition of claim 22 comprising Compound **3** of claim 2, mTOR inhibitor rapamycin and Bcl-2 inhibitor ABT-199.

28. The "three-in-one" pharmaceutical composition of claim 22 comprising Compound **5** of claim 2, mTOR inhibitor rapamycin and Bcl-2 inhibitor ABT-199.

29. A "three-in-one" pharmaceutical composition comprising a BTK inhibitor, a Bcl-2 inhibitor and a PI3K inhibitor.

30. The "three-in-one" pharmaceutical composition of claim 29 comprising BTK inhibitor ibrutinib, Bcl-2 inhibitor ABT-199 and PI3K inhibitor idelalisib.

31. The "three-in-one" pharmaceutical composition of claim 29 comprising BTK inhibitor Compound **3** of claim 2, Bcl-2 inhibitor ABT-199 and PI3K inhibitor idelalisib.

32. The "three-in-one" pharmaceutical composition of claim 29 comprising BTK inhibitor Compound **5** of claim 2, Bcl-2 inhibitor ABT-199 and PI3K inhibitor idelalisib.

33. The "three-in-one" pharmaceutical composition of claim 29 comprising BTK inhibitor ibrutinib, Bcl-2 inhibitor ABT-199 and PI3K inhibitor duvelisib.

34. The "three-in-one" pharmaceutical composition of claim 29 comprising BTK inhibitor Compound **3** of claim 2, Bcl-2 inhibitor ABT-199 and PI3K inhibitor duvelisib.

35. The "three-in-one" pharmaceutical composition of claim 29 comprising BTK inhibitor Compound **5** of claim 2, Bcl-2 inhibitor ABT-199 and PI3K inhibitor duvelisib.

36. A "three-in-one" pharmaceutical composition comprising a BTK inhibitor, an mTOR inhibitor and methotrexate.

37. The "three-in-one" pharmaceutical composition of claim 36 comprising BTK inhibitor ibrutinib, mTOR inhibitor everolimus and methotrexate.

38. The "three-in-one" pharmaceutical composition of claim 36 comprising Compound **3** of claim 2, mTOR inhibitor everolimus and methotrexate.

39. The "three-in-one" pharmaceutical composition of claim 36 comprising Compound **5** of claim 2, mTOR inhibitor everolimus and methotrexate.

40. The "three-in-one" pharmaceutical composition of claim 36 comprising BTK inhibitor ibrutinib, mTOR inhibitor rapamycin and methotrexate.

41. The "three-in-one" pharmaceutical composition of claim 36 comprising Compound **3** of claim 2, mTOR inhibitor rapamycin and methotrexate.

42. The "three-in-one" pharmaceutical composition of claim 36 comprising Compound **5** of claim 2, mTOR inhibitor rapamycin and methotrexate.

43. A "two-in-one" pharmaceutical composition comprising a BTK inhibitor and an mTOR inhibitor.

44. The "two-in-one" pharmaceutical composition of claim 43 comprising BTK inhibitor ibrutinib and mTOR inhibitor everolimus.

45. The "two-in-one" pharmaceutical composition of claim 43 comprising Compound **3** of claim 2 and mTOR inhibitor everolimus.

46. The "two-in-one" pharmaceutical composition of claim 43 comprising Compound **5** of claim 2 and mTOR inhibitor everolimus.

47. The "two-in-one" pharmaceutical composition of claim 43 comprising BTK inhibitor ibrutinib and mTOR inhibitor rapamycin.

48. The "two-in-one" pharmaceutical composition of claim 43 comprising Compound **3** of claim 2 and mTOR inhibitor rapamycin.

49. The "two-in-one" pharmaceutical composition of claim 43 comprising Compound **5** of claim 2 and mTOR inhibitor rapamycin.

50. A "two-in-one" pharmaceutical composition comprising a BTK inhibitor and a TOPK inhibitor.

51. The "two-in-one" pharmaceutical composition of claim 50 comprising BTK inhibitor ibrutinib and TOPK inhibitor OTS964.

52. The "two-in-one" pharmaceutical composition of claim 50 comprising Compound **3** of claim 2 and TOPK inhibitor OTS964.

53. The "two-in-one" pharmaceutical composition of claim 50 comprising Compound **5** of claim 2 and TOPK inhibitor OTS964.

54. A "two-in-one" pharmaceutical composition comprising a PI3K inhibitor and a TOPK inhibitor.

55. The "two-in-one" pharmaceutical composition of claim 54 comprising PI3K inhibitor idelalisib and TOPK inhibitor OTS964.

56. The "two-in-one" pharmaceutical composition of claim 54 comprising PI3K inhibitor duvelisib and TOPK inhibitor OTS964.

57. A "two-in-one" pharmaceutical composition comprising Compound 3 of claim 2 and PI3K inhibitor idelalisib.

58. A "two-in-one" pharmaceutical composition comprising Compound **5** of claim 2 and PI3K inhibitor idelalisib.

59. A "two-in-one" pharmaceutical composition comprising Compound **3** of claim 2 and PI3K inhibitor duvelisib.

60. A "two-in-one" pharmaceutical composition comprising Compound **5** of claim 2 and PI3K inhibitor duvelisib.

61. A "two-in-one" pharmaceutical composition comprising Compound **3** of claim 2 and Bcl-2 inhibitor ABT-199.

62. A "two-in-one" pharmaceutical composition comprising Compound **5** of claim 2 and Bcl-2 inhibitor ABT-199.

63. Use of a pharmaceutical composition of any one of claims 15 to 62 for the manufacture of a medicament for treating or inhibiting cancer or other diseases, wherein the cancer or other diseases include, but are not limited to, various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL) and multiple myeloma (MM)) and other diseases that would benefit from inhibition of BTK activity.

64. Use of a pharmaceutical composition of any one of claims 15 to 62 for the manufacture of a medicament for treating or inhibiting an autoimmune disease or disorder, wherein the autoimmune disease includes, but is not limited to, an organ-specific autoimmune disease such as chronic lymphocytic thyroiditis, hyperthyroidism, insulin-dependent diabetes mellitus, myasthenia gravis, chronic ulcerative colitis, pernicious anemia associated with chronic atrophic gastritis, Goodpasture's syndrome, pemphigus vulgaris, pemphigoid, primary biliary cirrhosis, multiple cerebrospinal sclerosis, acute idiopathic neuritis, etc.; a systemic autoimmune disease such as systemic lupus erythematosus, rheumatoid arthritis, systemic vasculitis, scleroderma, pemphigus, mixed connective tissue disease, autoimmune hemolytic anemia, autoimmune thyroid disease, ulcerative colitis, etc.

65. The use of claim 64, wherein the autoimmune disease is rheumatoid arthritis.

66. Use of a pharmaceutical composition of any one of claims 15 to 62 for the manufacture of a medicament for treating or inhibiting an immune disorder, wherein the immune disorder includes, but is not limited to, serum sickness, asthma, allergic rhinitis, drug allergy, etc.

67. Use of a pharmaceutical composition of any one of claims 15 to 62 for the manufacture of a medicament for treating or inhibiting an inflammatory disease or disorder, wherein the inflammatory disease includes, but is not limited to, keratitis, rhinitis, stomatitis, mumps, pharyngitis, tonsillitis, tracheitis, bronchitis, pneumonia, myocarditis, gastritis, gastroenteritis, cholecystitis, appendicitis, etc.

68. A method of using a pharmaceutical composition of any one of claims 15 to 62 for treating or inhibiting cancer or other diseases, wherein the cancer or other diseases include, but are not limited to, various B-cell malignancies (including small lymphocytic lymphoma (SLL), chronic lymphocytic leukemia (CLL), diffuse large B-cell lymphoma (DLBCL), Waldenstrom Macroglobulinemia, follicular lymphoma, mantle cell lymphoma (MCL) and multiple myeloma (MM)) and other diseases that would benefit from inhibition of BTK activity.

69. A method of using a pharmaceutical composition of any one of claims 15 to 62 for treating or inhibiting an autoimmune disease or disorder, wherein the autoimmune disease includes, but is not limited to, an organ-specific autoimmune disease such as chronic lymphocytic thyroiditis, hyperthyroidism, insulin-dependent diabetes mellitus, myasthenia gravis, chronic ulcerative colitis, pernicious anemia associated with chronic atrophic gastritis, Goodpasture's syndrome, pemphigus vulgaris, pemphigoid, primary biliary cirrhosis, multiple cerebrospinal sclerosis, acute idiopathic neuritis, etc.; a systemic autoimmune disease such as systemic lupus erythematosus, rheumatoid arthritis, psoriasis, systemic vasculitis, scleroderma, pemphigus, mixed connective tissue disease, autoimmune hemolytic anemia, autoimmune thyroid disease, ulcerative colitis, etc.

70. The method of claim 68, wherein the autoimmune disease is rheumatoid arthritis.

71. A method of using a pharmaceutical composition of any one of claims 15 to 62 for treating or inhibiting an immune disorder, wherein the immune disorder includes, but is not limited to, serum sickness, asthma, allergic rhinitis, drug allergy, etc.

72. A method of using a pharmaceutical composition of any one of claims 15 to 62 for treating or inhibiting an inflammatory disease or disorder, wherein the inflammatory disease includes, but is not limited to, keratitis, rhinitis, stomatitis, mumps, pharyngitis, tonsillitis, tracheitis, bronchitis, pneumonia, myocarditis, gastritis, gastroenteritis, cholecystitis, appendicitis, etc.
